# EUROPEAN PATENT APPLICATION

(11) **EP 2 341 349 A1**
(43) Date of publication of application: **06.07.2011**
(21) Application number: 10175828.2
(22) Date of filing: 03.05.2001
(51) Int. Cl.: G01N 33/58, C12Q 1/68, C09B 47/08

(54) **Methods for detection of multiple analytes**

(30) Priority: 04.05.2000 US 564230
(62) Divisional of application: 01933052.1
(71) Applicant: Siemens Healthcare Diagnostics Products GmbH, 35041 Marburg (DE)
(72) Inventor: Pease, John, Santa Clara, CA 95050 (US); Cromer, Remy, San Jose, CA 95130 (US); Patel, Rajesh, Fremont, CA 94539 (US); Kurn, Nurith, Palo Alto, CA 94306 (US); De Keczer, Steve, Saratoga, CA 95070 (US)
(74) Representative: Schweitzer, Klaus

(57) **Abstract**

Methods, compositions and kits are disclosed. The methods are directed to determining the presence or relative amounts of two or more components in a medium. A combination is provided comprising a medium suspected of containing the components, at least two sensitizer reagents and at least one reactive reagent activatable by singlet oxygen. The sensitizer reagents are capable of generating singlet oxygen and are distinguishable by wavelength of sensitization. The combination of sensitizer reagents and reactive reagents allows differential detection of the components. The sensitizer reagents are differentially activated. The amount of signal generated as a result of the activation of said reactive reagent is determined wherein the amount thereof is related to the amount of each of the components in the medium.

## Description

### BACKGROUND OF THE INVENTION

### 1. Field of the Invention.

This invention relates to methods, compositions and kits for detecting a number of different components in a single test medium.

The clinical diagnostic field has seen a broad expansion in recent years, both as to the variety of materials of interest that may be readily and accurately determined, as well as the methods for the determination. Convenient, reliable and non-hazardous means for detecting the presence of low concentrations of materials in liquids is desired. In clinical chemistry these materials may be present in body fluids in concentrations below 10⁻¹² molar. The difficulty of detecting low concentrations of these materials is enhanced by the relatively small sample sizes that can be utilized.

The need to determine multiple analytes in blood and other biological fluids has become increasingly apparent in many branches of medicine. In endocrinology the knowledge of plasma concentration of a number of different hormones is often required to resolve a diagnostic problem or a panel of markers for a given diagnosis where the ratios could assist in determining disease progression. An even more pressing need is evident in other areas such as allergy testing, the screening of transfused blood for viral contamination or genetic diagnosis.

Any one of a number of infectious agents may cause some pathological disease states. In other cases the diagnosis and assessment of disease states may be best evaluated by the measurement of a number of analytes in a sample, such as a panel of cytokines and chemokines, a panel of tissue specific disease markers, a panel of diagnostic antibodies and antigens and the like. Another example for the utility of simultaneous analysis of multi-analytes is the determination of the level of expression of a panel of genes in a given cell population or the simultaneous detection and quantification of multiple nucleic acid sequences in a single sample. Other benefits of simultaneous detection and quantification of multiple analytes are the potential increase in throughput of the analysis and the ability to incorporate internal controls to the test sample.

In other assays such as nucleic acid hybridization assays, there is need to detect and quantify specific target and positive control sequence in a single tube without time consuming separations and transfer steps. In principle, internal controls will eliminate the need for a standard curve. Amplification and detection in a single tube without opening the tube also overcomes contamination problems. In mutation analysis, the ability to measure two or more variants in a single tube would allow one to monitor quantitatively the appearance of mutant populations.

Most multi-analyte assays are heterogeneous, have poor sensitivity and poor dynamic range (2 to 100 fold difference in concentration of the analytes is determined) and some require the use of sophisticated instrumentation. A homogeneous assay that has higher sensitivity, large dynamic range (10³ to 10⁴ - fold difference in analyte concentration), and fewer and more stable reagents would increase the simplicity and reliability or multianalyte assays.

Luminescent compounds, such as fluorescent compounds and chemiluminescent compounds, find wide application in the assay field because of their ability to emit light. For this reason, luminescers have been utilized as labels in assays such as nucleic acid assays and immunoassays. For example, a member of a specific binding pair is conjugated to a luminescer and various protocols are employed. The luminescer conjugate can be partitioned between a solid phase and a liquid phase in relation to the amount of analyte in a sample suspected of containing the analyte. By measuring the luminescence of either of the phases, one can relate the level of luminescence observed to a concentration of the analyte in the sample.

Previously described systems for the simultaneous detection and quantification of multiple analytes in a single sample or multiples analysis are based on multiple reporter groups each corresponding to a specific analyte. Various labels have been used to produce distinguishable signals in multianalyte assays: (a) two different radioisotope labels, (b) two or more different fluorescent labels, (c) a fluorescent and a chemiluminescent label, (c) different lanthanide chelates where both lifetime and wavelength are measured, (e) an enzyme and an acridinium ester, (f) spatial resolution of different analytes, (g) different enzymes with sequential substrate additions, and (h) different acridinium esters that produce dioxetanones having different lifetimes.

In another approach a single reporter group is employed that reacts differently with various analytes to yield multiplicity of signals. When the analysis involves multiple signals in a single reaction mixture, the signals may be resolved based on wavelength difference and or differences in half-life of emission.

### 2. Brief Description of the Related Art.

U.S. Patent No. 5,656,207 (Woodhead, et al.) (Woodhead I) discloses a method for detecting or quantifying multiple analytes using labeling techniques.

Detecting or quantifying multiple analytes using labeling techniques is discussed by Woodhead, *et al.* (Woodhead II).

Compositions and methods for the simultaneous detection and quantification of multiple specific nucleic acid sequences is disclosed in U.S. Patent Nos. 5,827,656 (Nelson, et al.*)* (Nelson I) and 5,658,737 (Nelson, et al.*)* (Nelson II).

U.S. Patent No. 5,395,752 (Law, et al.*)* discloses long emission wavelength chemiluminescent compounds and their use in test assays.

Chandler, *et al*., discusses multiplexed analysis of clinical specimens, apparatus and method in PCT WO 97/14028.

A rapid, sensitive, multiplexed assay for the detection of viral nucleic acids using the FlowMetrix System is described by Smith, et al., in Clinical Chemistry (1998) 44(9):2054-2056.

Narang, *et al.,* discuss multianalyte detection using a capillary-based flow immunosensor (Analytical Biochemistry (1998) 255:13-19).

Rapid multiparameter analysis of cell simulation in mixed lymphocyte culture reactions is described by Traganos, et al., in The Journal of Histochemistry and Cytochemistry (1977) 25(7):881-887).

U.S. Patent No. 5,340,716 (Ullman, et al.) describes an assay method utilizing photoactivated chemiluminescent labels.

Photoactivatable chemiluminescent matrices are described in U.S. Patent No. 5,709,994 (Pease, et al.).

### SUMMARY OF THE INVENTION

One aspect of the present invention is a method for determining the presence or relative amounts of two or more components in a medium. A combination is provided comprising a medium suspected of containing the components and at least two sensitizer reagents and at least one reactive reagent activatable by the product of activated sensitizer reagent. The sensitizer reagents are distinguishable on the basis of activation. The combination of sensitizer reagent and reactive reagent allows differential detection of the components. The sensitizer reagents are differentially activated and the amount of signal generated corresponding to the activation of the reactive reagent by the product is measured. The amount of signal is related to the amount of each of the components in the medium.

Another embodiment of the present invention is a method for determining the presence or relative amounts of two or more components in a medium. A combination is provided that includes a medium suspected of containing the components. Also provided in the combination are at least two sensitizer reagents, which are capable of generating singlet oxygen and are distinguishable on the basis of activation. The combination also includes at least one reactive reagent activatable by singlet oxygen. The combination of sensitizer reagents and reactive reagents allows differential detection of the components. The sensitizer reagents are differentially activated and the amount of signal generated as a result of the activation of the reactive reagent is determined. The amount of signal is related to the amount of each of the components in the medium.

Another embodiment of the present invention is a method for determining the presence or relative amounts of two or more components in a medium. The method comprises providing in combination (1) a medium suspected of containing the components, (2) at least two sensitizer reagents, the sensitizer reagents being capable of generating singlet oxygen and being distinguishable by wavelength of sensitization, and (3) at least one reactive reagent activatable by singlet oxygen. A first specific binding pair (sbp) member is associated with each of the sensitizer reagents. The first sbp member is capable of binding to the component or to a second sbp member to form a complex related to the amount of the component. The sensitizer reagents are differentially activated. The amount of signal generated as a result of the activation of each of the reactive reagents is detected, the amount thereof being related to the amount of each of the components in the medium.

Another embodiment of the present invention is a homogeneous method for determining the presence or relative amounts of two or more components in a medium. A combination is provided comprising (1) a medium suspected of containing the components, (2) a photosensitizer for each of the components, and (3) chemiluminescent compositions activatable by singlet oxygen, each of which is associated with a second particle. The photosensitizer associated with a first particle is capable of generating singlet oxygen and has different wavelengths of sensitization. One or more of the chemiluminescent compositions are differentially detectable by different wavelengths of emission or by different rates of decay. The different wavelengths of sensitization for the photosensitizer and the different wavelengths of emission or different rates of decay of the chemiluminescent compositions result in the differential detection of each of the components. A first specific binding pair (sbp) member is associated with each of the photosensitizers. The first sbp member is capable of binding to the component or to a second sbp member to form a complex related to the amount of the component. The photosensitizers are differentially irradiated with light. The amount of luminescence generated by each of the chemiluminescent compositions is detected at a time after activation and at a wavelength corresponding to the rate of decay and wavelength of the luminescent emission, the amount thereof being related to the amount of each of the components in the medium.

Another embodiment of the present invention is a kit comprising in packaged combination a plurality of sensitizer reagents and at least one reactive reagent that is activatable by singlet oxygen. Each of the sensitizer reagents comprises (1) a sensitizer composition that is capable of generating singlet oxygen and is associated with a particle and (2) a member of a specific binding pair (sbp). Some portion or all of the sensitizer compositions have different wavelengths of sensitization.

Another embodiment of the present invention is a compound which is bis(trialkyl¹-silyl)silicon tetra-alkyl²-naphthalocyanine. An aspect of this embodiment is a method of making bis(tri-alkyl¹-silyl)silicon tetra-alkyl²-naphthalocyanine wherein the method comprises treating dihydroxysilicon tetra-alkyl²-naphthalocyanine with tri-alkyl¹-silylchloride.

### BRIEF DESCRIPTION OF THE DRAWINGS

Fig. 1 is a schematic depicting an assay scheme in accordance with the present invention.
Fig. 2 is a graph depicting standard curves.
Fig. 3 is a schematic depicting an assay scheme of the present invention.
Fig. 4 is a graph depicting standard curves.
Fig. 5 is a graph depicting standard curves.

### DESCRIPTION OF THE SPECIFIC EMBODIMENTS

The present invention permits quantitative detection of different analytes in an assay by the use of different sensitizer reagents that are differentially activatable usually by difference in wavelength of sensitization. Preferably, the sensitizer reagent is capable of generating singlet oxygen. A reactive reagent is also employed that is capable of being activated as a result of the activation of the sensitizer reagents, usually capable of activation by singlet oxygen. Whereas previous chemiluminescence or fluorescence based, homogeneous methods for multiplex analysis are based on resolution of specific signals based on rates of decay and/or wavelength of emission, the present method is based on distinction of specific signals based on the illumination wavelengths for sensitization of the specific sensitizers. The reactive reagents may be chemiluminescers, fluorescers, photoactive indicator precursors and the like or combinations thereof. Other signal producing acceptors that are activated by reaction with singlet oxygen may also be used in the multiplex analysis of the present invention. Combination of multiple sensitizers and suitable multiple acceptors allow further multiplexing of the analysis.

Before proceeding further with a description of the specific embodiments of the present invention, a number of terms will be defined and described in detail.

Component - component of interest; the compound or composition to be detected. The component may be an analyte, a reference compound, a control compound, a calibrator, and the like.

Analyte -- the analyte is usually comprised of a member of a specific binding pair (sbp) and may be a ligand, which is monovalent (monoepitopic) or polyvalent (polyepitopic), usually antigenic or haptenic, and is a single compound or plurality of compounds which share at least one common epitopic or determinant site. The analyte can be a part of a cell such as a bacterium or a cell bearing a blood group antigen such as A, B, D, etc., or an HLA antigen or the analyte may be a microorganism, e.g., bacterium, fungus, protozoan, or virus.

The polyvalent ligand analytes will normally be poly(amino acids), i.e., polypeptides and proteins, polysaccharides, nucleic acids, and combinations thereof. Such combinations include components of bacteria, viruses, chromosomes, genes, mitochondria, nuclei, cell membranes and the like.

For the most part, the polyepitopic ligand analytes to which the subject invention can be applied will have a molecular weight of at least about 5,000, more usually at least about 10,000. In the poly(amino acid) category, the poly(amino acids) of interest will generally be from about 5,000 to 5,000,000 molecular weight, more usually from about 20,000 to 1,000,000 molecular weight; among the hormones of interest, the molecular weights will usually range from about 5,000 to 60,000 molecular weight.

A wide variety of proteins may be considered as to the family of proteins having similar structural features, proteins having particular biological functions, proteins related to specific microorganisms, particularly disease causing microorganisms, etc. Such proteins include, for example, immunoglobulins, cytokines, enzymes, hormones, cancer antigens, nutritional markers, tissue specific antigens, etc. Such proteins include, by way of illustration and not limitation, protamines, histones, albumins, globulins, scleroproteins, phosphoproteins, mucoproteins, chromoproteins, lipoproteins, nucleoproteins, glycoproteins, T-cell receptors, proteoglycans, HLA, unclassified proteins, e.g., somatotropin, prolactin, insulin, pepsin, proteins found in human plasma, blood clotting factors, protein hormones such as, e.g., follicle-stimulating hormone, luteinizing hormone, luteotropin, prolactin, chorionic gonadotropin, tissue hormones, cytokines, cancer antigens such as, e.g., PSA, CEA, a-fetoprotein, acid phosphatase, CA19.9 and CA125, tissue specific antigens, such as, e.g., alkaline phosphatase, myoglobin, CPK-MB and calcitonin, and peptide hormones. Other polymeric materials of interest are mucopolysaccharides and polysaccharides.

The monoepitopic ligand analytes will generally be from about 100 to 2,000 molecular weight, more usually from 125 to 1,000 molecular weight. The analytes include drugs, metabolites, pesticides, pollutants, and the like. Included among drugs of interest are the alkaloids. Among the alkaloids are morphine alkaloids, which includes morphine, codeine, heroin, dextromethorphan, their derivatives and metabolites; cocaine alkaloids, which include cocaine and benzyl ecgonine, their derivatives and metabolites; ergot alkaloids, which include the diethylamide of lysergic acid; steroid alkaloids; iminazoyl alkaloids; quinazoline alkaloids; isoquinoline alkaloids; quinoline alkaloids, which include quinine and quinidine; diterpene alkaloids, their derivatives and metabolites.

The next group of drugs includes steroids, which includes the estrogens, androgens, andreocortical steroids, bile acids, cardiotonic glycosides and aglycones, which includes digoxin and digoxigenin, saponins and sapogenins, their derivatives and metabolites. Also included are the steroid mimetic substances, such as diethylstilbestrol.

The next group of drugs is lactams having from 5 to 6 annular members, which include the barbituates, e.g., phenobarbital and secobarbital, diphenylhydantoin, primidone, ethosuximide, and their metabolites.

The next group of drugs is aminoalkylbenzenes, with alkyl of from 2 to 3 carbon atoms, which includes the amphetamines; catecholamines, which includes ephedrine, L-dopa, epinephrine; narceine; papaverine; and metabolites of the above.

The next group of drugs is benzheterocyclics, which include oxazepam, chlorpromazine, tegretol, their derivatives and metabolites, the heterocyclic rings being azepines, diazepines and phenothiazines.

The next group of drugs is purines, which includes theophylline, caffeine, their metabolites and derivatives.

The next group of drugs includes those derived from marijuana, which includes cannabinol and tetrahydrocannabinol.

The next group of drugs is the hormones such as thyroxine, cortisol, triiodothyronine, testosterone, estradiol, estrone, progestrone, polypeptides such as angiotensin, LHRH, and immunosuppresants such as cyclosporin, FK506, mycophenolic acid, and so forth.

The next group of drugs includes the vitamins such as A, B, e.g. B12, C, D, E and K, folic acid, thiamine.

The next group of drugs is prostaglandins, which differ by the degree and sites of hydroxylation and unsaturation.

The next group of drugs is the tricyclic antidepressants, which include imipramine, dismethylimipramine, amitriptyline, nortriptyline, protriptyline, trimipramine, chlomipramine, doxepine, and desmethyldoxepin,

The next group of drugs is the anti-neoplastics, which include methotrexate.

The next group of drugs is antibiotics, which include penicillin, Chloromycetin, actinomycetin, tetracycline, Terramycin, the metabolites and derivatives.

The next group of drugs is the nucleosides and nucleotides, which include ATP, NAD, FMN, adenosine, guanosine, thymidine, and cytidine with their appropriate sugar and phosphate substituents.

The next group of drugs is miscellaneous individual drugs which include methadone, meprobamate, serotonin, meperidine, lidocaine, procainamide, acetylprocainamide, propranolol, griseofulvin, valproic acid, butyrophenones, antihistamines, chloramphenicol, anticholinergic drugs, such as atropine, their metabolites and derivatives.

Metabolites related to diseased states include spermine, galactose, phenylpyruvic acid, and porphyrin Type 1.

The next group of drugs is aminoglycosides, such as gentamicin, kanamicin, tobramycin, and amikacin.

Among pesticides of interest are polyhalogenated biphenyls, phosphate esters, thiophosphates, carbamates, polyhalogenated sulfenamides, their metabolites and derivatives.

For receptor analytes, the molecular weights will generally range from 10,000 to 2X10⁸, more usually from 10,000 to 10⁶. For immunoglobulins, IgA, IgG, IgE and IgM, the molecular weights will generally vary from about 160,000 to about 10⁶. Enzymes will normally range from about 10,000 to 1,000,000 in molecular weight. Natural receptors vary widely, generally being at least about 25,000 molecular weight and may be 10⁶ or higher molecular weight, including such materials as avidin, DNA, RNA, thyroxine binding globulin, thyroxine binding prealbumin, transcortin, etc.

The term analyte further includes polynucleotide analytes such as those polynucleotides defined below. These include m-RNA, r-RNA, t-RNA, DNA, DNA-RNA duplexes, etc. The term analyte also includes receptors that are polynucleotide binding agents, such as, for example, restriction enzymes, activators, repressors, nucleases, polymerases, histones, repair enzymes, chemotherapeutic agents, and the like.

The analyte may be a molecule found directly in a sample such as biological tissue, including body fluids, from a host. The sample can be examined directly or may be pretreated to render the analyte more readily detectable. Furthermore, the analyte of interest may be determined by detecting an agent probative of the analyte of interest such as a specific binding pair member complementary to the analyte of interest, whose presence will be detected only when the analyte of interest is present in a sample. Thus, the agent probative of the analyte becomes the analyte that is detected in an assay. The biological tissue includes excised tissue from an organ or other body part of a host and body fluids, for example, urine, blood, plasma, serum, saliva, semen, stool, sputum, cerebral spinal fluid, tears, mucus, and the like.

Sensitizer - a molecule, usually a compound, that is activatable to form a product, such as singlet oxygen, which is capable of activating a reactive reagent. The sensitizers useful in the present invention are differentially activatable such as by having different wavelengths of activation or different modes of activation. The sensitizer can be photoactivatable (e.g., dyes and aromatic compounds) or chemi-activatable (e.g., enzymes and metal salts). Preferably, the sensitizer is a photosensitizer. However, other sensitizers include, by way of example and not limitation, other substances and compositions that can produce singlet oxygen with or, less preferably, without activation by an external light source. Thus, for example, molybdate (MoO₄⁼) salts and chloroperoxidase and myeloperoxidase plus bromide or chloride ion (Kanofsky, J. Biol. Chem. (1983) 259:5596) have been shown to catalyze the conversion of hydrogen peroxide to singlet oxygen and water. Either of these compositions can, for example, be included in particles to which is bound an sbp member and used in the assay method wherein hydrogen peroxide is included as an ancillary reagent, chloroperoxidase is bound to a surface and molybdate is incorporated in the aqueous phase of a liposome. Also included as sensitizers within the scope of the invention are compounds that are not true sensitizers but which on excitation by heat, light, ionizing radiation, or chemical activation will release a molecule of singlet oxygen. The best known members of this class of compounds includes the endoperoxides such as 1,4-biscarboxyethyl-1,4-naphthalene endoperoxide, 9,10-diphenylanthracene-9,10-endoperoxide and 5,6,11,12-tetraphenyl naphthalene 5,12-endoperoxide. Heating or direct absorption of light by these compounds releases singlet oxygen.

Photosensitizer - a sensitizer for generation of singlet oxygen usually by excitation with light. When excited by light the photosensitizer is usually a compound comprised of covalently bonded atoms, usually with multiple conjugated double or triple bonds. The compound should absorb light in the wavelength range of about 200 to about 1100 nm, usually, about 300 to about 1000 nm, preferably, about 450 to about 950 nm, with an extinction coefficient at its absorbance maximum greater than about 1000 M⁻¹ cm⁻¹, preferably, greater than about 50,000 M⁻¹ cm⁻¹, more preferably, about 100,000 M⁻¹ cm⁻¹, at the excitation wavelength. Preferably, the sensitizer is excited at long, distinct wavelengths of about 400 to about 1000 nm, more preferably, about 600 to about 800 nm.

Photosensitizers that are to be excited by light will be relatively photostable and, preferably, will not react efficiently with singlet oxygen. Several structural features are present in most useful photosensitizers. Most photosensitizers have at least one and frequently three or more conjugated double or triple bonds held in a rigid, frequently aromatic structure. They will frequently contain at least one group that accelerates intersystem crossing such as a carbonyl or imine group or a heavy atom selected from rows 3-6 of the periodic table, especially iodine or bromine, or they may have extended aromatic structures. Typical photosensitizers include acetone, benzophenone, 9-thioxanthone, eosin, 9,10-dibromoanthracene, methylene blue, metallo-porphyrins, such as hematoporphyrin, phthalocyanines, naphthocyanines, chlorophylls, rose bengal, buckminsterfullerene, etc., and derivatives of these compounds having substituents of 1 to 50 atoms for rendering such compounds more lipophilic or more hydrophilic and/or as attaching groups for attachment, for example, to an sbp member. Examples of other photosensitizers that may be utilized in the present invention are those that have the above properties and are enumerated in N. F. Turro, "Molecular Photochemistry" page 132, W. A. Benjamin Inc., N.Y. 1965.

Examples of long wavelength sensitizer dyes that may be employed in the present invention are long wavelength sensitizer dyes useful for photodynamic therapy (Woehrie, et al., In Proc. SPIE-Int. Soc. Opt. Eng. 1996, 319-327, and Macromol. Symp. 1996, 105, 127-138), metallotexaphyrins absorbing at 730 to 770 nm (Harriman, et al., J. Chem. Soc. Chem. Commun., 1989, 314) and texaphyrins (J.L. Sessler and S.J. Wegnorn, 1997, Tetrahedron Organic Chemistry Series Vol. 15.

The photosensitizers are preferably relatively non-polar to assure dissolution into a lipophilic member when the photosensitizer is incorporated in an oil droplet, liposome, latex particle, etc.

Reactive reagent - a molecule, usually a compound, that is activatable by a product produced by a sensitizer. The reactive reagent may be, for example, a chemiluminescent composition, a photoactivatable indicator precursor, a photoactivatable chromophore, a photoactivatable fluorophor, and the like.

Chemiluminescent composition - a chemiluminescer; a compound that undergoes a detectable change upon reaction with the product of the sensitizer. Examples of chemiluminescers, by way of illustration and not limitation, are olefins capable of reacting with singlet oxygen, e.g., to form hydroperoxides or dioxetanes, stable dioxetanes that can be activated with base or an enzyme, acetylenes that can react with singlet oxygen to form diketones, hydrazones or hydrazides that are activated with a peroxide and that can form azo compounds or azo carbonyls such as luminol, chemiluminescent enzyme substrates such as luciferin, aromatic compounds that can form endoperoxides, etc.

The chemiluminescent compound can produce any detectable signal upon reaction with singlet oxygen. The signal will usually be initiated by and/or detected as electromagnetic radiation and will preferably be luminescence such as chemiluminescence, fluorescence, electroluminescence or phosphorescence.

Olefins capable of reaction with singlet oxygen -- a typical reaction of olefins with singlet oxygen is 2 + 2 addition to form a dioxetane. Suitable olefins usually have no saturated C-H group attached to an olefinic carbon except unreactive bridgehead carbons and will preferably have one or more electron donating groups directly attached to the olefinic carbon or in conjugation with the olefin. Dioxetanes can dissociate spontaneously or by heating with spontaneous chemiluminescence, or the carbonyl groups that are formed can be formed as part of a fluorescent group or be capable of undergoing subsequent reactions that lead to a fluorescent molecule. Alternatively, this dissociation reaction can lead to separation of a quenching group from a fundamentally fluorescent group that thereby regains its fluorescent property.

Another type of reaction of singlet oxygen with olefins is 4 + 2 cycloaddition with dienes, usually aromatic compounds such as naphthalenes, anthracenes, oxazoles, furans, indoles, and the like. Such a reaction leads initially to an endoperoxide. In some cases endoperoxides can rearrange to active esters or anhydrides that are capable of reaction with a suitably placed group to provide a lactone or lactam that can be fluorescent. Alternatively, the endoperoxides may oxidize a fluorescent or chemiluminescent compound precursor. Endoperoxides can also dissociate spontaneously or on heating with chemiluminescent emission or oxidize a fluorescent leuco dye.

Still another type of reaction of singlet oxygen with olefins is the "ene" reaction that produces an allylhydroperoxide. Suitable olefins have a reactive saturated C-H attached to an olefinic carbon. This product can react with an active ester in the same molecule to form a dioxetanone that can spontaneously or by heating dissociate with chemiluminescent emission.

In general, olefins of interest are those that undergo a chemical reaction upon reaction with singlet oxygen to form a metastable reaction product, usually a dioxetane or endoperoxide, which is capable of decomposition with the simultaneous or subsequent emission of light, usually within the wavelength range of 250 to 1200 nm. Preferred are electron rich olefins usually containing electron-donating groups. Exemplary of such electron rich olefins are enol ethers, enamines, 9-alkylidene-N-alkylacridans, arylvinylethers, 1,4-dioxenes, 1,4-thioxenes, 1,4-oxazines, arylimidazoles, 9-alkylidene-xanthanes and lucigenin.

The luminescence produced upon reaction of the olefins of interest with singlet oxygen will preferably be at wavelengths above 300 nanometers, preferably above 500 nanometers, and more preferably above 550 nm. Compounds that absorb light at wavelengths beyond the region where the sample components contribute significantly to light absorption will be of particular use in the present invention. The absorbance of serum drops off rapidly above 500 nm and becomes insignificant above 600 nm. Luminescence above 550 nm is of particular interest. However, luminescence at shorter wavelengths is useful when interference absorbance of the sample is absent. Preferably, the chemiluminescent olefins will absorb light at less than about 400 nm to permit convenient handling in room light without the risk of inadvertently producing singlet oxygen by photosensitization.

Examples of suitable electron rich chemiluminescent olefins are set forth in U.S. Patent No. 5,709,994, the relevant disclosure of which is incorporated herein by reference. Such olefins generally have an electron-donating group in conjugation with the olefin.

The dioxetanes may be luminescent alone or in conjunction with a fluorescent energy acceptor. Enol ethers are examples of such olefins. Frequently, the enol ether compounds will have at least one aryl group bound to the olefinic carbons where the aryl ring is substituted with an electron donating group at a position that increases the reactivity of the olefin to singlet oxygen and/or imparts fluorescence to the product of dissociation of the resultant dioxetane. The electron-donating group can be, for example, hydroxyl, alkoxy, disubstituted amino, alkyl thio, furyl, pyryl, etc. Preferably, the enol ethers have an electron-donating group bound directly to an olefinic carbon.

Enamines are another example of such olefins. In general, useful enamines will be governed by the rules set forth above for enol ethers.

Another family of chemiluminescers is the 2,4,5-triphenylimidazoles, with lophine as the common name for the parent product. Chemiluminescent analogs include para-dimethylamino and -methoxy substituents.

Other chemiluminescent olefins that satisfy the requirements given above may be found in European Patent Application No. 0,345,776.

Photoactive indicator precursor - a molecule, usually a compound, that reacts with singlet oxygen to form photoactive indicators or to form a compound that can react with an auxiliary compound that is thereupon converted to a photoactive indicator. There are several types of reactions of singlet oxygen that can give compounds that will lead to a photoactive indicator compound. The type of reaction that is employed and the choice of the photoactive indicator that is desired provides a guide to the structures of the photoactive indicator precursors and any auxiliary compounds used in the present invention.

The photoactive indicator precursor preferably undergoes a reaction with singlet oxygen that is very rapid, usually at least about 10⁴ to about 10⁶ sec⁻¹, preferably at least about 10⁶ to about 10⁸ sec⁻¹, more preferably, greater than about 10⁹ sec⁻¹. When the initial product of the reaction is an intermediate that reacts to give the photoactive precursor, the intermediate preferably has a lifetime that is short relative to the desired time between forming singlet oxygen and detecting the fluorescence emitted from the photoactive indicator upon exposure to light. For simultaneous singlet oxygen generation and fluorescence detection the lifetime usually is about 10⁻³ to about 10 sec, preferably, about 10-³. When generation of singlet oxygen and fluorescence detection are sequential the lifetime may vary from about 10⁻³ to about 30 minutes or more, preferably less than about 1 sec to about 60 sec.

Higher rates of reaction of singlet oxygen are achieved by providing singlet oxygen reactive groups in the photoactive indicator precursor that are electron rich. These groups are usually an olefin or acetylene, hydrazine and hydroxylamine derivatives, selenides and tellurides but are not limited to these groups. For example, tellurides have been found to be particularly useful because they react rapidly with singlet oxygen to produce an olefin. The reaction rate depends on the electron availability (oxidation potential) of the tellurium. For example, electron-donating groups on an aryl ring substituent on the tellurium atom can increase the rate. Changing from tellurium to selenium (the next lower chalcogenide) decreases the rate, but increases the oxidation stability of the molecule.

When the photoactive indicator precursor contains a hydrazine or hydrazide, reaction with singlet oxygen can produce a double bond. For example, singlet oxygen can convert hydrazides directly into fluorescent photoactive indicators.

The oxidation potential of a hydrazine is an important factor in providing a high rate of reaction. Electron withdrawing groups such as an acyl group (e.g., as in a hydrazide) slow the reaction although acyl hydrazides and diacyl hydrazides can still be used as photoactive indicator precursors in the present invention. When the reaction is insufficiently rapid it can often be accelerated in the presence of a base. For example, 3-aminophthaloyl hydrazide forms an anion in the presence of strong base that is electron rich and can react rapidly with singlet oxygen to form 3-aminophthalate as the photoactive indicator. However, the hydroxyl ion cannot be used as a base when the suspendible particles contain the photoactive indicator precursor within a hydrophobic matrix. Hydrophilic particles such as agarose can be used instead to permit access to the hydroxyl ion. Usually the photoactive indicator precursor will be covalently bound to the suspendible particle when the particle is hydrophilic. Still another example of a useful singlet oxygen reaction is the reaction with electron rich olefins such as those described in European Published Patent Application No. 0 515 194. Two fundamental types of reactions are described. One of these is the "ene" reaction, which shifts the position of a double bond and produces a hydroperoxide. The double bond shift can cause two auxachromic groups in the photoactive indicator precursor to come into conjugation and thus produce a fluorescent photoactive indicator.

Other photoactive indicator precursors react with singlet oxygen to form hydroperoxides, which can react internally with an oxidizable group to give a fluorescent photoactive indicator. Alternatively, a hydroperoxide formed by reaction of singlet oxygen with a photoactive indicator precursor, such as 1,3-diphenylpropene, can serve to oxidize the leuco form of a dye that is present as an auxiliary compound so as to form a fluorescent photoactive indicator. The hydroperoxide can also oxygenate a group V element in an auxiliary compound to cause it to act as an electron donating quencher of an associated fluorescent group. The auxiliary compound could alternatively have a selenium or tellurium atom that could react with a hydroperoxide to produce an intermediate that could undergo subsequent elimination to form a fluorescent photoactive indicator.

Alternatively, the photoactive indicator precursor will react slowly or not at all with singlet oxygen but will react with a hydroperoxide reaction product of singlet oxygen and an auxiliary molecule. For example, in the following reaction, the auxiliary compound is reacted with singlet oxygen to form a hydroperoxide, which is then reacted with a photoactive indicator precursor to form a fluorescent photoactive indicator.

In each of these examples the auxiliary compound and the photoactive indicator precursor may be covalently linked. In such an occurrence, the resulting molecule is referred to as a photoactive indicator precursor.

The structure of the photoactive indicator precursor therefore depends on the particular singlet oxygen reaction that is to be employed and it will usually be designed to assure that any subsequent reactions initiated by reaction with singlet oxygen that are required to produce a photoactive indicator will proceed relatively rapidly. Additionally, the structure of the photoactive indicator precursor will lead to the formation of a photoactive indicator that has the desired absorption and emission wavelengths, and has relatively high fluorescent quantum yields, preferably greater than 0.1, more preferably greater than 0.4, and a high extinction coefficient at the desired excitation wavelength, preferably greater than 1000 M⁻¹ cm⁻¹, more preferably greater than 10,000 M⁻¹ cm⁻¹.

Other classes of photoactive indicator precursors can also be used in the present invention. For example, compounds that chemiluminesce on reaction with singlet oxygen are frequently converted to fluorescent products which can serve as photoactive indicators of the present invention.

"Photoactive indicator" refers to a molecule which, following absorption of light of wavelengths of 250 to 1100 nm, preferably 300 to 950 nm, emits light by fluorescence or phosphorescence, preferably by fluorescence, or transfers it excitation energy to an acceptor molecule which thereupon emits light by fluorescence or phosphorescence. Preferably the emission quantum yield will be high, usually at least 0.1, preferably at least 0.4, more preferably greater than 0.7 and the extinction coefficient of the absorption maximum will usually be greater than 5000 M⁻¹ cm⁻¹. Photoactive indicators are typically fluorescent compounds, such as fluorescent brighteners, which typically absorb light between 300 and 400 nanometers and emit between 400 and 500 nanometers; xanthenes such as rhodamine and fluorescein; coumarins such as umbelliferone; aromatic amines such as dansyl; squarate dyes; benzofurans; cyanines, merocyanines, rare earth chelates, and the like. Photoactive indicators that are phosphorescent include porphyrins, phthalocyanines, polyaromatic compounds such as pyrene, anthracene and acenaphthene. Photoactive indicators also include chromenes. Photoactive indicators that can transfer energy to an acceptor molecule will usually absorb at 250 to 550 nm. Such acceptor molecules are luminescent and can include any of the above mentioned fluorescent and phosphorescent photoactive indicators.

A more detailed discussion of photoactive indicator precursors and photoactive indicators is set forth in U.S. Patent No. 5,807,675 (Davalian, et al.*),* the relevant portions of which are incorporated herein by reference.

Fluorescent energy acceptor - a chemiluminescent compound may have a fluorescent energy acceptor in close proximity thereto. Typically, the fluorescent energy acceptor is a chromophore having substantial absorption higher than 310 nm, normally higher than 350 nm, and preferably higher than about 400 nm. The width of the emission band at half peak height will usually be less than 100 nm, preferably less than 50 nm, more preferably, less than 25 nm. The choice of the fluorescent energy acceptor will be governed primarily by the particular chemiluminescer and the desired wavelength and lifetime of emission. The fluorescent energy acceptor should be capable of absorbing light emitted by the chemiluminescer. Preferably, the absorption maximum of the fluorescent energy acceptor should be at similar wavelength as the emission maximum of the chemiluminescer. A high extinction coefficient is desirable, usually in excess of 10, preferably in excess of 10³, and particularly preferred in excess of 10⁴. The fluorescent energy acceptor preferably has a high fluorescence quantum yield, usually at least 0.1, preferably greater than 0.4.

Preferred fluorescent energy acceptors are long wavelength, preferably hydrophobic, emitters including polycyclic aromatic hydrocarbons such as anthracenes, e.g., bisphenylethynylanthracene; coumarins; naphthacenes; phthalocyanines; squaraines, bis-(4-dimethlyaminophenyl)squaraine; porphyrins; polyacetylenes, oxazine dyes; rare earth chelates, especially, Eu, Tb and Sm, and the like. In general these dyes act as acceptors in energy transfer processes and preferably have high fluorescent quantum yields and do not react rapidly with singlet oxygen. They can be incorporated into matrices together with the chemiluminescer. Hydrophilic fluorescent dyes may also be used, particularly cyanine dyes, xanthenes such as fluorescein and Texas red, and umbelliferones.

A number of different molecules useful as the fluorescent energy acceptor are described by Ullman, et al. in U.S. Patent Nos. 4,261,968, 4,174,384, 4,199,559 and 3,996,345, at columns 8 and 9, the relevant portions of which are incorporated herein by reference.

The fluorescent energy acceptor may be formed as a result of a compound that reacts with singlet oxygen to form a fluorescent compound or a compound that can react with an auxiliary compound that is thereupon converted to a fluorescent compound. The fluorescent energy acceptor may be incorporated as part of a compound that also includes the chemiluminescer. For example, the fluorescent energy acceptor may include a metal chelate of a rare earth metal such as, e.g., europium, samarium, tellurium and the like. These materials are particularly attractive because of their sharp band of luminescence.

As used herein, the term "associated with" includes association through covalent or non-covalent binding or through incorporation into, such as incorporation into a matrix.

Matrix -- a support comprised of an organic or inorganic, solid or fluid, water insoluble material, which may be transparent or partially transparent. The primary requirement of the matrix having a reactive reagent incorporated therein is that it permits the diffusion of singlet oxygen therein at least to the proximate location of the incorporated reactive reagent. The matrix can have any of a number of shapes, such as particle, including bead, film, membrane, tube, well, strip, rod, and the like. The surface of the matrix is, preferably, hydrophilic or capable of being rendered hydrophilic. The body of the matrix is, preferably, hydrophobic. The matrix may be suspendable in the medium in which it is employed. Examples of suspendable matrices in accordance with the present invention, by way of illustration and not limitation, are polymeric materials such as latex, lipid bilayers, oil droplets, cells and hydrogels. Other matrix compositions include polymers, such as nitrocellulose, cellulose acetate, poly (vinyl chloride), polyacrylamide, polyacrylate, polyethylene, polypropylene, poly(4-methylbutene), polystyrene, polymethacrylate, poly(ethylene terephthalate), nylon, poly(vinyl butyrate), etc.; either used by themselves or in conjunction with other materials.

Binding of sbp members to the matrix may be direct or indirect, covalent or non-covalent and can be accomplished by well-known techniques, commonly available in the literature. See, for example, "Immobilized Enzymes," Ichiro Chibata, Halsted Press, New York (1978) and Cuatrecasas, J. Biol. Chem., 245:3059 (1970).

The surface of the matrix will usually be polyfunctional or be capable of being polyfunctionalized or be capable of binding to an sbp member, or the like, through covalent or specific or non-specific non-covalent interactions. Such binding is indirect where non-covalent interactions are used and is direct where covalent interactions are employed. A wide variety of functional groups are available or can be incorporated. Functional groups include carboxylic acids, aldehydes, amino groups, cyano groups, ethylene groups, hydroxyl groups, mercapto groups and the like. The manner of linking a wide variety of compounds to surfaces is well known and is amply illustrated in the literature (see above). The length of a linking group to the oligonucleotide or sbp member may vary widely, depending upon the nature of the compound being linked, the effect of the distance between the compound being linked and the surface on the specific binding properties and the like.

The reactive reagent may be incorporated into the matrix either during or after the preparation of the matrix. The reactive reagent is usually chosen to dissolve in the matrix but may be covalently attached to the matrix. The reactive reagents, when not covalently attached, are usually hydrophobic to reduce their ability to dissociate from the matrix. In general the matrix composition is chosen so as to favor association of the reactive reagent with the matrix.

The amount of reactive reagent associated with or incorporated into the matrix in the compositions of the invention depends upon a number of factors such as the nature of the reactive reagent, e.g., chemiluminescer, fluorescent energy acceptor, photoactive indicator precursor, etc., and the matrix and the intended use of the resulting reagent. The reactive reagent is present in the matrix in an amount necessary to maximize the signal produced in accordance with the invention, i.e., to provide the highest signal to background in an assay. Generally, the amount of reactive reagent is determined empirically and is usually about from 10⁻⁸ to 1M, preferably, from 10⁻⁵ to 10⁻² M, more preferably, 10⁻³ to 10⁻¹ M.

In general, an sbp member is present in from about 0.5 to about 100, more usually about 1 to about 90, frequently from about 5 to about 80 and preferably from about 50 to about 100 mole percent of the molecules present on the surface of the matrix. The particular amount of sbp member is also dependent on a number of factors and is usually best determined empirically.

Particles -- particles of at least about 20 nm and not more than about 20 microns, usually at least about 40 nm and less than about 10 microns, preferably from about 0.10 to 2.0 microns diameter, normally having a volume of less than 1 picoliter. The particle may have any density, but preferably of a density approximating water, generally from about 0.7 to about 1.5g/ml. The particles may or may not have a charge, and when they are charged, they are preferably negative. The particles may be solid (e.g., comprised of organic and inorganic polymers or latex), oil droplets (e.g., hydrocarbon, fluorocarbon, silicon fluid), or vesicles (e.g., synthetic such as phospholipid or natural such as cells and organelles).

The solid particles are normally polymers, either addition or condensation polymers, which are readily dispersible in the assay medium. The solid particles will also be adsorptive or functionalizable so as to bind or attach at their surface, either directly or indirectly, an sbp member and to incorporate within their volume a reactive reagent.

The solid particles can be comprised of polystyrene, polyacrylamide, homopolymers and copolymers of derivatives of acrylate and methacrylate, particularly esters and amides, silicones and the like. The particles may be bound or attached to an sbp member as described above.

Oil droplets -- are water-immiscible fluid particles comprised of a lipophilic compound coated and stabilized with an emulsifier that is an amphiphilic molecule such as, for example, phospholipids, sphingomyelin, albumin and the like that exist as a suspension in an aqueous solution, i.e. an emulsion. Emulsions comprising oil droplets can be made in accordance with conventional procedures by combining the appropriate lipophilic compounds with a surfactant, anionic, cationic or nonionic, where the surfactant is present in from about 0.1 to 5, more usually from about 0.1 to 2 weight percent of the mixture and subjecting the mixture in an aqueous medium to agitation, such as sonication or vortexing. Illustrative lipophilic compounds include hydrocarbon oils, halocarbons including fluorocarbons, alkyl phthalates, trialkyl phosphates, triglycerides, etc.

Sbp members, and where appropriate the reactive reagent and sensitizer reagents, can be bound to the droplets in a number of ways. As described by Giaever, *supra,* the particular sbp member, e.g., a proteinaceous sbp member, can be coated on the droplets by introducing an excess of the sbp member into the aqueous medium prior to or after the emulsification step. Washing steps are desirable to remove excess sbp member. Functionalization of the oil introduces functionalities described above for linking to sbp members.

A sensitizer reagent or a reactive reagent may be chosen to be soluble in the oil phase of the oil droplet. When the oil is a fluorocarbon, a fluorinated sensitizer reagent or a reactive reagent is often more soluble than the corresponding unfluorinated derivation.

Liposomes -- microvesicles comprised of one or more lipid bilayers having approximately spherical shape and one of the preferred materials for use in the present invention. The liposomes have a diameter that is at least about 20 nm and not more than about 20 microns, usually at least about 40 nm and less than about 10 microns. Preferably, the diameter of the liposomes will be less than about two microns so as to limit settling or floatation.

Phospholipids employed in preparing particles utilizable in the present invention can be any phospholipid or phospholipid mixture found in natural membranes including lecithin, or synthetic glyceryl phosphate diesters of saturated or unsaturated 12-carbon or 24-carbon linear fatty acids wherein the phosphate can be present as a monoester, or as an ester of a polar alcohol such as ethanolamine, choline, inositol, serine, glycerol and the like. Particularly preferred phospholipids include L-a-palmitoyl oleoyl-phosphatidylcholine (POPC), palmitoyl oleoylphosphatidyl-glycerol (POPG), L-α-dioleoylphosphatidylglycerol, L-α (dioleoyl)-phosphatidyl ethanolamine (DOPE) and L-α-(dioleoyl)-phosphatidyl-(4-(N-maleimidomethyl)-cyclohexane-1-carboxyamido)ethanol (DOPE-MCC).

For use in the present invention the liposomes should be capable of binding to an sbp member and be capable of having a sensitizer reagent or reactive reagent associated with either the aqueous or the nonaqueous phase.

Liposomes may be produced by a variety of methods including hydration and mechanical dispersion of dried phospholipid or phospholipid substitute in an aqueous solution. Liposomes prepared in this manner have a variety of dimensions, compositions and behaviors. One method of reducing the heterogeneity and inconsistency of behavior of mechanically dispersed liposomes is by sonication. Such a method decreases the average liposome size. Alternatively, extrusion is usable as a final step during the production of the liposomes. U.S. Patent 4,529,561 discloses a method of extruding liposomes under pressure through a uniform pore-size membrane to improve size uniformity.

Preparation of liposomes containing a sensitizer reagent or a reactive reagent dissolved in the lipid bilayer can be carried out in a variety of methods, including a method described by Olsen, et al., Biochemica et Biophysica Acta, 557(9), 1979.

Latex particles -- "Latex" signifies a particulate water suspendable water insoluble polymeric material usually having particle dimensions of 20 nm to 20 mm, more preferably 100 to 1000 nm in diameter. The latex is frequently a substituted polyethylene such as polystyrene-butadiene, polyacrylamide polystyrene, polystyrene with amino groups, poly-acrylic acid, polymethacrylic acid, acrylonitrile-butadiene, styrene copolymers, polyvinyl acetate-acrylate, polyvinyl pyridine, vinyl-chloride acrylate copolymers, and the like. Non-crosslinked polymers of styrene and carboxylated styrene or styrene functionalized with other active groups such as amino, hydroxyl, halo and the like are preferred. Frequently, copolymers of substituted styrenes with dienes such as butadiene will be used.

The association of the sensitizer reagent or reactive reagent with latex particles utilized in the present invention may involve incorporation during formation of the particles by polymerization but will usually involve incorporation into preformed particles, usually by noncovalent dissolution into the particles. Usually, a solution of the reagent will be employed.

An sbp member or member of the label reagent may be physically adsorbed on the surface of the latex particle or may be covalently bonded or attached to the particle in a manner similar to that discussed above with respect to other matrices.

Member of a specific binding pair ("sbp member") -- one of two different molecules, having an area on the surface or in a cavity which specifically binds to and is thereby defined as complementary with a particular spatial and polar organization of the other molecule. The members of the specific binding pair are referred to as ligand and receptor (antiligand). These will usually be members of an immunological pair such as antigen-antibody, although other specific binding pairs such as biotin-avidin, hormones-hormone receptors, nucleic acid duplexes, IgG-protein A, polynucleotide pairs such as DNA-DNA, DNA-RNA, and the like are not immunological pairs but are included in the invention and the definition of sbp member.

Polynucleotide -- a compound or composition which is a polymeric nucleotide having in the natural state about 50 to 500,000 or more nucleotides and having in the isolated state about 15 to 50,000 or more nucleotides, usually about 15 to 20,000 nucleotides, more frequently 15 to 10,000 nucleotides. The polynucleotide includes nucleic acids from any source in purified or unpurified form, naturally occurring or synthetically produced, including DNA (dsDNA and ssDNA) and RNA, usually DNA, and may be t-RNA, m-RNA, r-RNA, mitochondrial DNA and RNA, chloroplast DNA and RNA, DNA-RNA hybrids, or mixtures thereof, genes, chromosomes, plasmids, the genomes of biological material such as microorganisms, e.g., bacteria, yeasts, viruses, viroids, molds, fungi, plants, animals, humans, and fragments thereof, and the like.

Ligand -- any organic compound for which a receptor naturally exists or can be prepared.

Ligand analog -- a modified ligand, an organic radical or analyte analog, usually of a molecular weight greater than 100, which can compete with the analogous ligand for a receptor, the modification providing means to join a ligand analog to another molecule. The ligand analog will usually differ from the ligand by more than replacement of a hydrogen with a bond that links the ligand analog to a hub or label, but need not. The ligand analog can bind to the receptor in a manner similar to the ligand. The analog could be, for example, an antibody directed against the idiotype of an antibody to the ligand.

Receptor ("antiligand")--any compound or composition capable of recognizing a particular spatial and polar organization of a molecule, e.g., epitopic or determinant site. Illustrative receptors include naturally occurring receptors, e.g., thyroxine binding globulin, antibodies, enzymes, Fab fragments, lectins, nucleic acids, protein A, complement component C1 q, and the like.

Specific binding -- the specific recognition of one of two different molecules for the other compared to substantially less recognition of other molecules. Generally, the molecules have areas on their surfaces or in cavities giving rise to specific recognition between the two molecules. Exemplary of specific binding are antibody-antigen interactions, enzyme - substrate interactions, polynucleotide interactions, and so forth.

Non-specific binding -- non-covalent binding between molecules that is relatively independent of specific surface structures. Non-specific binding may result from several factors including hydrophobic interactions between molecules.

Antibody -- an immunoglobulin that specifically binds to and is thereby defined as complementary with a particular spatial and polar organization of another molecule. The antibody can be monoclonal or polyclonal and can be prepared by techniques that are well known in the art such as immunization of a host and collection of sera (polyclonal) or by preparing continuous hybrid cell lines and collecting the secreted protein (monoclonal), or by cloning and expressing nucleotide sequences or mutagenized versions thereof coding at least for the amino acid sequences required for specific binding of natural antibodies. Antibodies may include a complete immunoglobulin or fragment thereof, which immunoglobulins include the various classes and isotypes, such as IgA, IgD, IgE, IgG1, IgG2a, IgG2b and IgG3, IgM, etc. Fragments thereof may include Fab, Fv and F(ab')₂, Fab', and the like. In addition, aggregates, polymers, and conjugates of immunoglobulins or their fragments can be used where appropriate so long as binding affinity for a particular molecule is maintained.

Substituted -- means that a hydrogen atom of a molecule has been replaced by another atom, which may be a single atom such as a halogen, etc., or part of a group of atoms forming a functionality such as a substituent having from 1 to 50 atoms (other than the requisite hydrogen atoms necessary to satisfy the valencies of such atoms), which atoms are independently selected from the group consisting of carbon, oxygen, nitrogen, sulfur, halogen (chlorine, bromine, iodine, fluorine) and phosphorus, and which may or may not be bound to one or more metal atoms.

Electron-donating group -- a substituent which, when bound to a molecule, is capable of polarizing the molecule such that the electron-donating group becomes electron poor and positively charged relative to another portion of the molecule, i.e., has reduced electron density. Such groups include, by way of illustration and not limitation, amines, ethers, thioethers, phosphines, hydroxy, oxyanions, mercaptans and their anions, sulfides, etc.

A substituent having from 1 to 50 atoms (other than the requisite hydrogen atoms necessary to satisfy the valencies of such atoms), which atoms are independently selected from the group consisting of carbon, oxygen, nitrogen, sulfur, halogen and phosphorus -- an organic radical; the organic radical has 1 to 50 atoms other than the requisite number of hydrogen atoms necessary to satisfy the valencies of the atoms in the radical. Generally, the predominant atom is carbon (C) but may also be oxygen (O), nitrogen (N), sulfur (S), phosphorus (P), wherein the O, N, S, or P, if present, are bound to carbon or one or more of each other or to hydrogen or a metal atom to form various functional groups, such as, for example, carboxyl groups (carboxylic acids), hydroxyl groups (alcohols), mercapto groups (thiols), carboxamides, carbamates, carboxylic acid esters, sulfonic acids, sulfonic acid esters, phosphoric acids, phosphoric acid esters, ureas, carbamates, phosphoramides, sulfonamides, ethers, sulfides, thioethers, olefins, acetylenes, amines, ketones, aldehydes and nitriles, and alkyl, alkylidine, aryl, aralkyl, and alkyl, aryl, and aralkyl substituted with one or more of the aforementioned functional groups, e.g., phenyl, naphthyl, phenanthryl, m-methoxyphenyl, dimethylamino, trityl, methoxy, N-morpholino and may be taken together to form a ring such as, for example, adamantyl, N-methyacridanylide, xanthanylidine, 1-(3,4-benzo-5-hydrofurylidene), and the like.

Linking group -- a group involved in the covalent linkage between molecules. The linking group will vary depending upon the nature of the molecules, i.e., the sensitizer, reactive reagent, matrix, sbp member or molecule associated with, or part of, a particle being linked. Functional groups that are normally present or are introduced on a matrix or an sbp member will be employed for linking these materials.

For the most part, carbonyl functionalities will find use, both oxocarbonyl, e.g., aldehyde, and non-oxocarbonyl (including nitrogen and sulfur analogs) e.g., carboxy, amidine, amidate, thiocarboxy and thionocarboxy.

Alternative functionalities of oxo include active halogen, diazo, mercapto, olefin, particularly activated olefin, amino, phosphoro and the like. A description of linking groups may be found in U.S. Patent No. 3,817,837, which disclosure is incorporated herein by reference in its entirety.

The linking groups may vary from a bond to a chain of from 1 to 100 atoms, usually from about 1 to 70 atoms, preferably 1 to 50 atoms more preferably 1 to 20 atoms, each independently selected from the group normally consisting of carbon, oxygen, sulfur, nitrogen, halogen and phosphorous. The number of heteroatoms in the linking groups will normally range from about 0 to 20, usually from about 1 to 15, more preferably 2 to 6. The atoms in the chain may be substituted with atoms other than hydrogen in a manner similar to that described above for the substituent having from 1 to 50 atoms. As a general rule, the length of a particular linking group can be selected arbitrarily to provide for convenience of synthesis and the incorporation of any desired group such as an energy acceptor, fluorophor, group for analysis of intersystem crossing such as a heavy atom, and the like. The linking groups may be aliphatic or aromatic, although with diazo groups, aromatic groups will usually be involved.

When heteroatoms are present, oxygen will normally be present as oxo or oxy, bonded to carbon, sulfur, nitrogen or phosphorous, nitrogen will normally be present as nitro, nitroso or amino, normally bonded to carbon, oxygen, sulfur or phosphorous; sulfur would be analogous to oxygen; while phosphorous will be bonded to carbon, sulfur, oxygen or nitrogen, usually as phosphonate and phosphate mono- or diester.

Common functionalities in forming a covalent bond between the linking group and the molecule to be conjugated are alkylamine, amidine, thioamide, ether, urea, thiourea, guanidine, azo, thioether and carboxylate, sulfonate, and phosphate esters, amides and thioesters.

For the most part, when a linking group will have a non-oxocarbonyl group including nitrogen and sulfur analogs, a phosphate group, an amino group, alkylating agent such as halo or tosylalkyl, oxy (hydroxyl or the sulfur analog, mercapto) oxocarbonyl (e.g., aldehyde or ketone), or active olefin such as a vinyl sulfone or α-, β-unsaturated ester. These functionalities will be linked to amine groups, carboxyl groups, active olefins, alkylating agents, e.g., bromoacetyl. Where an amine and carboxylic acid or its nitrogen derivative or phosphoric acid are linked, amides, amidines and phosphoramides will be formed. Where mercaptan and activated olefin are linked, thioethers will be formed. Where a mercaptan and an alkylating agent are linked, thioethers will be formed. Where aldehyde and an amine are linked under reducing conditions, an alkylamine will be formed. Where a carboxylic acid or phosphate acid and an alcohol are linked, esters will be formed.

A group or functionality imparting hydrophilicity or water solubility -- is a hydrophilic functionality, which increases wettability of solids with water and the solubility in water of compounds to which it is bound. Such functional group or functionality can be a substituent having 1 to 50 or more atoms and can include a group having a sulfonate, sulfate, phosphate, amidine, phosphonate, carboxylate, hydroxyl particularly polyols, amine, ether, amide, and the like. Illustrative functional groups are carboxyalkyl, sulfonoxyalkyl, CONHOCH₂COOH, CO-(glucosamine), sugars, dextran, cyclodextrin, SO₂NHCH₂COOH, SO₃H, CONHCH₂CH₂SO₃H, PO₃H₂, OPO₃H₂, hydroxyl, carboxyl, ketone, and combinations thereof. Most of the above functionalities can also be utilized as attaching groups, which permit attachment of an sbp member or the like to a particulate composition comprised of the label.

A group or functionality imparting lipophilicity or lipid solubility -- is a lipophilic functionality, which decreases the wettability of surfaces by water and the solubility in water of compounds to which it is bound. Such functional group or functionality can contain 1 to 50 or more atoms, usually carbon atoms substituted with hydrogen or halogen and can include alkyl, alkylidene, aryl and aralkyl. The lipophilic group or functionality will normally have one to six straight or branched chain aliphatic groups of at least 6 carbon atoms, more usually at least 10 carbon atoms, and preferably at least 12 carbon atoms, usually not more than 30 carbon atoms. The aliphatic group may be bonded to rings of from 5 to 6 members, which may be alicyclic, heterocyclic, or aromatic. Lipophilic groups may be bonded to a label or other substance to increase its solubility in a non-aqueous matrix.

Ancillary Materials -- Various ancillary materials will frequently be employed in an assay in accordance with the present invention. For example, buffers will normally be present in the assay medium, as well as stabilizers for the assay medium and the assay components. Frequently, in addition to these additives, proteins may be included, such as albumins; organic solvents such as formamide; quaternary ammonium salts; polyanions such as dextran sulfate; surfactants, particularly non-ionic surfactants; binding enhancers, e.g., polyalkylene glycols; or the like.

Wholly or partially sequentially -- when the sample and various agents utilized in the present invention are combined other than concomitantly (simultaneously), one or more may be combined with one or more of the remaining agents to form a subcombination. Each subcombination can then be subjected to one or more steps of the present method. Thus, each of the subcombinations can be incubated under conditions to achieve one or more of the desired results.

As mentioned above, the present method is directed to the determination of the presence or relative amounts of two or more components in a medium. A combination is provided comprising a medium suspected of containing the components and at lease two sensitizer reagents and at least one reactive reagent that is capable of reacting with the product of the activated sensitizer reagent. Preferably, the sensitizer reagent is capable of generating singlet oxygen. The interaction of each of the sensitizer reagents with a reactive reagent corresponds with, and thereby permits detection of, each respective component. The sensitizer reagents are differentially activated and the amount of signal is measured for each particular interaction. The amount of signal is related to the amount of each of the components in the medium. The reactive reagent(s) may be associated with a matrix by being incorporated therein or attached thereto. Furthermore, the sensitizer reagents may be associated with a matrix by being incorporated therein or attached thereto. One sensitizer reagent and one reactive reagent may be associated with the same matrix in a manner similar to that discussed in U.S. Patent No. 5,709,994, *supra.*

The assay is usually carried out in an aqueous buffered medium at a moderate pH, generally that which provides optimum assay sensitivity. The aqueous medium may be solely water or may include from 0.01 to 80 or more volume percent of a cosolvent. The pH for the medium will usually be in the range of about 4 to 13, more usually in the range of about 5 to 10, and preferably in the range of about 6.5 to 9.5. The pH is generally selected to achieve optimum assay sensitivity and specificity. Among the factors that must be considered are the pH dependence of the rates of the reactions involved, the binding of binding members and the minimization of non-specific binding, and so forth.

Various buffers may be used to achieve the desired pH and maintain the pH during the determination. Illustrative buffers include borate, phosphate, carbonate, tris, barbital and the like. The particular buffer employed is not critical to this invention, but in an individual assay one or another buffer may be preferred.

Moderate temperatures are normally employed for carrying out the assay and usually constant temperature, preferably, room temperature, during the period of the measurement. Incubation temperatures will normally range from about 5° to 99°C, usually from about 15° to 70°C, more usually 20 to 45°C. Temperatures during measurements will generally range from about 10° to 70°C, more usually from about 20° to 45°C, more usually 20° to 25°C.

In some instances an activated reactive reagent, e.g., an activated chemiluminescer, may require heating up to 100°C in order to decay to produce luminescence because the product of its reaction is relatively stable at ambient temperatures. Relatively stable dioxetanes can be formed, for example, by reaction of singlet oxygen with adamantylidenes (see, e.g., McCapra, supra) and relatively stable endoperoxides can be formed by reaction of singlet oxygen with 1,4-disubstituted naphthalenes and anthracenes (see, e.g., N.J. Turro, Modern Molecular Photochemistry (1978) Benjamin Cummings Publishing Co. page 594). In both circumstances above, the stable materials will undergo decay upon heating, usually, at a temperature of less than 200°C, preferably about 50 to 100°C. Such heating causes the rapid decomposition of the singlet oxygen/olefin adduct and, thus, the emission of light occurs over a short period of time. The use of this approach may be desirable when separate signals from different chemiluminescers are difficult to fully resolve by lifetime and wavelength.

The concentration of components to be detected will generally vary from about 10⁻⁵ to 10⁻¹⁷ M, more usually from about 10⁻⁶ to 10⁻¹⁴ M. Considerations, such as whether the assay is qualitative, semiquantitative or quantitative, the particular detection technique and the nature and concentration of the components of interest will normally determine the concentrations of the various reagents.

While the concentrations of the various reagents in the assay medium will generally be determined by the concentration range of interest of the components to be detected, the final concentration of each of the reagents will normally be determined empirically to optimize the sensitivity of the assay over the range. That is, a variation in concentration of the components to be detected that is of significance should provide an accurately measurable signal difference.

As mentioned above, one or both of the sensitizer reagents and reactive reagents may comprise a matrix, preferably in the form of particles, with which the sensitizer or reactive reagent is, or sensitizer and reactive reagent are, associated. In this embodiment the matrix has the sensitizer or reactive reagent or both incorporated therein and/or bound to its surface. The amount of the sensitizer or reactive reagent associated with the matrix is usually about up to about 20% of the weight of the matrix, more usually about 0.01 to about 20% of the weight of the matrix. Where one or more fluorescent energy acceptors are employed in conjunction with the reactive reagent, the fluorescent energy acceptor is usually about 10⁻⁷ to about 10⁻¹ M, preferably about 10⁻⁵ to about 10⁻² M.

While the order of addition may be varied widely, there will be certain preferences depending on the nature of the assay. The simplest order of addition is to add all the materials simultaneously. Alternatively, the reagents can be combined wholly or partially sequentially. One or more incubation steps may be involved after the reagents are combined, generally ranging from about 5 seconds to about 24 hours, usually, 30 seconds to 6 hours, more usually from about 2 minutes to 1 hour.

The primary control of the differential activation in the present method relates to the nature of the sensitizer. Light activated sensitizers generally are chosen on the basis of wavelength of activation. Structural characteristics that contribute to different wavelength of activation include electron donating and/or electron withdrawing groups, extent of conjugation within the molecule, planarity of the molecule, bond strain, metal ligands, presence of hetero atoms in the molecule and so forth. Other characteristics that contribute to different wavelength of activation include pH, solvent polarity, molecular complexes, and energy transfer from, for example, a fluorophor. When using energy transfer mechanism, one may chose the same sensitizer component in conjunction with various fluorophors for modulation of the illumination wavelength for photo activation. This will allow for increased multiplexing. The efficiency of singlet oxygen generation is also a factor in the choice of a sensitizer.

For light activated sensitizers the medium is irradiated with light to activate the sensitizer. Irradiation is generally carried out by means of an incandescent lamp such as quartz halogen or mercury halogen, light emitting diodes, solid state and gas lasers. The wavelengths of activation are discussed above.

Chemi-activated sensitizers are chosen on the basis of time of activation, which in turn relates to the type of activation. Chemi-activated sensitizers include compounds that have a chemically labile bond to a moiety, which prevents the compound from performing its function as a sensitizer. Such a labile bond may be to a moiety such as, for example, an ester, amide, acetal, and the like. The compound is chemi-activated by treatment with a chemical such as, for example, an acid or a base, that destroys the labile bond and renders the compound capable of performing as a sensitizer in the present invention. For chemi-activated sensitizers the medium generally is treated with the appropriate chemical reagent for activating the sensitizer. Usually, this involves adding the chemical reagent to the medium.

Enzymatic activation of a molecule is also included within the scope of the present invention. Removal of a group by an enzyme may activate a compound to become a photosensitizer. Photolabile protecting/masking groups may also be useful. The use of multiple photolabile groups in conjunction with a single sensitizer group increases the multiplexing capability.

As mentioned above, different sensitizers may be used in conjunction with different reactive reagents to achieve further multiplexing. Chemiluminescent compounds may be differentiated on the basis of wavelength of emission and/or rate of decay. Structural features that contribute to a delay in luminescence are discussed by Schaap, *supra,* and McCapra, *supra,* wherein the relevant portions of these references are incorporated herein by reference. Another factor that allows for control of the time to luminescence is the composition of the matrix such as the particle. In general, when the matrix is composed of a non-polar material in which the chemiluminescer is dissolved, decay times are increased in relation to polar materials. Another factor that may be used to control the rate of luminescence is temperature. In general, increasing the temperature will decrease the decay time.

The nature of the signal generating compound, e.g., chemiluminescer, fluorescer, fluorescent energy acceptor, and the like, determines the manner of measuring the signal. Light produced in the method can be measured visually, photographically, actinometrically, spectrophotometrically or by any other convenient means to determine the amount thereof, which is related to the amount of each component in the medium. Light emitted may be measured while the signal producing reagent is in contact with the assay medium, for example, by means of a luminometer or a photosensitive material.

One particular application of the methods and compositions of the invention is a method for determining the presence or relative amounts of a plurality of analytes, each of which is a member of a specific binding pair (sbp). A combination is provided comprising a medium suspected of containing a plurality of analytes, a plurality of photosensitizers and one or more reactive reagents. The number of combinations of photosensitizers and reactive reagents are sufficient to differentiate each of the analytes in the medium. For example, a different photosensitizer may be used for each analyte in conjunction with a single reactive reagent such as a chemiluminescer. On the other hand, the number of photosensitizers may be less than the total number of analytes to be differentiated. In this case more than one chemiluminescer may be used where these agents are distinguishable by wavelength of emission and/or rate of decay. Each photosensitizer reagent comprises a first sbp member bound to a particle with which the photosensitizer is associated. Each first sbp member is capable of binding to the analyte or to a second sbp member to form a complex related to the amount of the respective analyte. The combination is incubated in a medium under conditions sufficient to allow the sbp members to bind to the analytes or to respective second sbp members. The medium is differentially irradiated with light. The amount of luminescent emission generated by each of the reactive reagents is detected at a time after irradiation corresponding to the irradiation of a particular photosensitizer. Where multiple reactive reagents are employed, consideration is also given to the wavelength of emission and the time of emission from the respective reactive reagents. The amount of each measured luminescent emission is then related to the amount of each analyte in the medium.

In an alternative embodiment in a method for determining the presence or relative amounts of a plurality of analytes, each of which is a member of a specific binding pair (sbp), a combination is provided comprising a medium suspected of containing a plurality of analytes, a plurality of photosensitizers and one or more reactive reagents. As mentioned above, the number of combinations of photosensitizers and reactive reagents are sufficient to differentiate each of the analytes in the medium. Each reagent comprises a first sbp member bound to a particle with which the photosensitizer and reactive reagent are associated. Each first sbp member is capable of binding to the analyte or to a second sbp member to form a complex related to the amount of the respective analyte. The combination is incubated in a medium under conditions sufficient to allow the sbp members to bind to the analytes or to respective second sbp members. Unbound reagents are separated from reagents that are bound to respective analytes. This may be accomplished by standard techniques such as the use of a support to which is attached a binding reagent, which binds to all analytes in the medium that are bound by respective particle reagents. After separation of the unbound reagents from the support such as by washing, the bound reagents are differentially irradiated with light. The amount of luminescent emission generated by each of the reactive reagents is detected at a time after irradiation corresponding to the irradiation of a particular photosensitizer. Where multiple reactive reagents are employed, consideration is also given to the wavelength of emission and the time of emission from the respective reactive reagents. The amount of each measured luminescent emission is then related to the amount of each analyte in the medium. In this approach the sensitizer reagent combined with a reactive reagent in the same matrix functions merely as a signal generator.

The method and compositions of the invention may be adapted to most assays involving sbp members such as ligand-receptor, e.g., antigen-antibody reactions, polynucleotide binding assays, and so forth. The assays are usually homogeneous or heterogeneous, preferably homogeneous, including competitive and sandwich. In a specific binding assay, the sample may be pretreated, if necessary, to remove unwanted materials or to render the analyte detectable.

As mentioned previously, the first sbp member above is capable of binding to the analyte or to a second sbp member capable of binding to the analyte. When the second sbp member is also capable of binding to the analyte, a sandwich assay protocol can result. The immunological reaction for a sandwich type assay usually involves an sbp member, e.g., an antibody, that is complementary to the analyte, a second sbp member, e.g., antibody, that is also complementary to the analyte and bound to the particulate matrix, and the sample of interest.

One of the sbp members alternatively can be analogous to the analyte, in which case a competitive assay protocol can result. The immunological reaction for a competitive protocol usually involves an sbp member that is complementary to the analyte and an sbp member that is analogous to, usually a derivative of, the analyte. One of these sbp members will be associated with the matrix.

In one type of assay, a sample suspected of containing an analyte, which is an sbp member and the other assay reagents are combined and incubated. The medium is then irradiated at appropriate wavelengths corresponding to the number of photosensitizers employed. The medium is differentially examined for the presence of emission, usually by measuring the amount of light emitted, which is related to the amount of each analyte in the sample. This approach is a homogeneous assay where a separation step is not employed. Alternatively, a particulate or non-particulate matrix may be used, which, after combining the assay reagents and incubating, may be separated from the liquid phase, and either the solid phase or the liquid phase may then be irradiated differentially and examined differentially for the presence of emission.

Another example of an assay protocol is described next by way of example and not limitation. A chemiluminescent compound is associated with a particle, which is attached to a specific binding reagent (for example: antibody, oligonucleotide, receptor, etc.) that is complementary to the analyte. A sensitizer particle is attached to a second specific binding reagent that is complementary to the analyte. There is one sensitizer particle reagent for each separate analyte. In a sandwich assay format the analyte brings both the sensitizer particle and chemiluminescer particle in close proximity. Activation of sensitizer particles with light results in the formation of singlet oxygen, which is channeled to the particle label reagent. Usually, the sensitizer reagents are activated differentially and the resulting light emitted by each chemiluminescent composition is detected and measured sequentially.

By judicious choice of sensitizer reagents with different wavelengths of activation or different modes of activation and of reactive reagents with different lifetimes and different emission maxima, assays may be carried out with high sensitivity and large dynamic range. Sensitivity and precision of assays may be optimized by attention to reagent preparation such as coating of reagents on the surface of a matrix and the like. Thus, simultaneous detection and quantitation of a plurality of components in a sample can be conducted. As mentioned above, the reagents used in the present invention are most effective when activatable by singlet oxygen. The signals can be deconvoluted by difference in wavelength or mode of activation in conjunction with difference in wavelength and/or time of emission.

The foregoing compositions and assays are provided by way of illustration and not limitation to enable one skilled in the art to appreciate the scope of the present invention and to practice the invention without undue experimentation. It will be appreciated that the choice of components, e.g., analytes, label reagents, particles, other reagents and reaction conditions will be suggested to those skilled in the art in view of the disclosure herein and the examples that follow.

Another aspect of the present invention relates to kits useful for conveniently performing an assay method of the invention for determining the presence or relative amounts of two or more components in a medium. To enhance the versatility of the subject invention, the reagents can be provided in packaged combination, in the same or separate containers, so that the ratio of the reagents provides for substantial optimization of the method and assay. The reagents may each be in separate containers or various reagents can be combined in one or more containers depending on the cross-reactivity and stability of the reagents.

A kit of the present invention comprises in packaged combination a plurality of sensitizer reagents, each comprising (1) a sensitizer reagent that is capable of generating singlet oxygen and (2) a member of a specific binding pair (sbp). At least a portion of the sensitizer reagents has different wavelengths of sensitization. The sensitizer may be activated to produce singlet oxygen and may be a photosensitizer. A kit further comprises at least one reactive reagent that is activatable by singlet oxygen. The kit may further comprise multiple reactive reagents wherein one or more of said multiple reactive reagents are differentially detectable. The reactive reagent may be associated with an sbp member that is capable of binding with said component and may further be associated with a particle. The sensitizer reagent may be associated with a particle.

The kit can further include other separately packaged reagents for conducting an assay such as enzyme substrates, additional sbp members, ancillary reagents and so forth.

The relative amounts of the various reagents in the kits can be varied widely to provide for concentrations of the reagents that substantially optimize the reactions that need to occur during the present method and to further substantially optimize the sensitivity of the assay. Under appropriate circumstances one or more of the reagents in the kit can be provided as a dry powder, usually lyophilized, including excipients, which on dissolution will provide for a reagent solution having the appropriate concentrations for performing a method or assay in accordance with the present invention. The kit can further include a written description of a method in accordance with the present invention as described above.

As mentioned above another embodiment of the present invention is a compound which is bis(tri-alkyl¹-silyl)silicon tetra-alkyl²-naphthalocyanine. In a particular embodiment, by way of example and not limitation, alkyl¹ is hexyl and alkyl² is butyl and the compound is bis(tri-n-hexylsilyl)silicon tetra-t-butyl-naphthalocyanine (see below, section entitled Synthesis of naphthalocyanine sensitizer). Alkyl¹ and alkyl² are independently an alkyl group, which is a group comprising carbon and hydrogen, usually, about 1 to about 30 carbon atoms, preferably, about 4 to 20 carbon atoms. The alkyl group may be a straight or branched chain.

An aspect of this embodiment is a method of making bis(tri-alkyl¹-silyl)silicon tetra-alkyl²-naphthalocyanine. The method was a modification of a method disclosed in a thesis by J. Sounik, "Synthesis and Characterization of Group IV Naphthalocyanines," Case Western Reserve, 1988. In the present method dihydroxysilicon tetra-alkyl²-naphthalocyanine is treated with tri-alkyl¹-silylchloride. In a specific example, by way of illustration and not limitation, the method is directed to making bis(tri-n-hexylsilyl)silicon tetra-t-butyl-naphthalocyanine. In this specific embodiment, the method comprises treating dihydroxysilicon tetra-t-butyl-naphthalocyanine with tri-n-hexylsilylchloride (see below, section entitled Synthesis of naphthalocyanine sensitizer).

Briefly, the synthesis of bis(tri-n-hexylsilyl)silicon tetra-t-butyl-naphthalocyanine involves the preparation of 6-t-butyl-diiminobenz(f)isoindoline by treating 6-t-butyl-2,3-dicyanonaphthalene with strong base such as, for example, sodium methoxide, potassium methoxide, and so forth in a suitable solvent such as methanol, ethanol, and the like, followed by treatment with NH₃ gas for a period of about 0.5 to about 10 hours at a temperature of about 15°C to about 80°C. Next, dichlorosilicon tetra-t-butyl-naphthalocyanine (t-bu₄-NcCl₂) is prepared from the 6-t-butyl-diiminobenzisoindoline prepared as described above. The latter compound is dissolved in a suitable organic solvent such as, e.g., quinoline and the like, and silicon tetrachloride is added. The mixture is heated at a temperature of about 30°C to about 200°C for a period of about 15 minutes to about 4 hours. Next, dihydroxysilicon tetra-t-butyl-naphthalocyanine (t-bu₄-Nc(OH)₂) is prepared from dichlorosilicon tetra-t-butyl-naphthalocyanine (t-bu₄-NcCl₂) by treatment with, for example, concentrated sulfuric acid, and the like. The solution is generally stirred at about 50° to about 80° C for a period of about 2 to about 6 hours. After washing and drying, the product of the above reaction is treated with strong base such as, e.g., concentrated NH₄OH and the like.

Preparation of bis(tri-n-hexylsilyl)silicon tetra-t-butyl-naphthalocyanine (t-bu₄-Nc[hex₃Si]₂) is carried out by dissolving t-bu₄-Nc(OH)₂, prepared as described above, in a suitable organic solvent such as, for example, pyridine and the like, and combining with a solution of tri-n-hexylsilylchloride in pyridine. Usually, the reaction is carried out under an inert atmosphere such as, e.g., argon, nitrogen, and the like at a temperature of about 100° to about 130° C for a period of about 0.5 to about 4 hours. The products of any of the above reactions may be purified by well-known techniques such as chromatography, recrystallization, and the like.

### EXAMPLES

The invention is demonstrated further by the following illustrative examples. Parts and percentages recited herein are by weight unless otherwise specified. Temperatures are in degrees centigrade (°C). The following preparations and examples illustrate the invention but are not intended to limit its scope.

Melting points were determined on a Hoover capillary apparatus and are uncorrected. 'HNMR spectra were recorded on a Brucker WP-250 MHz or Brucker WP-300 MHz NMR spectrometer. Chemical shifts were reported in parts per million (δ 0.0). NMR multiplicities are recorded by use of the following abbreviations: s, singlet; d, doublet; t, triplet; m, multiplet; Hz, hertz. Infrared spectra were recorded on a Perkin-Elmer 2971R spectrometer. Desorption chemical ionization (C.I.) and electron ionization (E.I.) were done on a Varian-MAT 311A, double focusing high-resolution mass spectrometer. A Finnigan TSQ-70 or MAT-8230 was used for fast atom bombardment mass spectra (FAB/LSIMS). Some mass spectra were obtained from the UC Berkeley Mass Spectrometry Laboratory. Particle sizing was run on a NICOMP Submicron Particle Sizer, Model 370. Ultracentrifugation was done on a Du Pont Instruments Sorvall RC 5B Refrigerated Superspeed Centrifuge. UV spectra were run on a Hewlett Packard model 8452A Diode Array Spectrophotometer. Fluorescence measurements were done on a Spex fluorolog spectrophotometer or a Perkin Elmer 650-40 spectrophotometer. Chemiluminescence measurements were performed on a custom built chemiluminometer fitted with 675 and 780 nm lasers as light sources.

Toluene and THF were distilled from sodium over argon and diisopropylethylamine was dried over 3A sieves. 2-ethoxyethanol was from Aldrich Chemical Co. and was redistilled under vacuum. Other solvents were used without purification, and most reactions were carried out under argon. Silica gel used for flash chromatography was 230-400 mesh ASTM, purchased from Scientific Products while TLC preparative plates (1000µ) and analytical plates (250µ) were purchased from Analtech.

1-Chloro 9,10- bis(phenylethynyl) anthracene (1-Cl-BPEA) and rubrene (5,6,11,12-tetraphenyl naphthacene) were purchased from Aldrich Chemical Co. Rubrene was recrystallized from methylene chloride and stored at 4°C in a brown bottle prior to use. Heptadecylbenzene was purchased from Pfaltz and Bauer, Inc. (Waterbury, CT).

Carboxylate-modified polystyrene (latex) particles were purchased from Seradyn, Inc. The particles were 203 ± 4.0 nM. The carboxyl parking area was 49.5 angstroms squared (0.09 milliequivalents/g). Solids were 10% (100 mg/ml).

Dextran T-500 was from Pharmacia (Piscataway, NJ). SIAX and TCEP were purchased from Molecular Probes, Inc. (Eugene, OR). HBR-1 was from Scantibodies. Gentamycin sulfate was from Life technologies. Kathon preservative and other common reagents used in the preparation of buffers, etc., were obtained from Sigma, (St Louis, MO).

DNA oligonucleotides, probes, and linkers were purchased from Oligos Etc. Inc. (Wilsonville, OR) and received as lyophylized powders that were dissolved in sterile water and stored frozen until used.

The following abbreviations have the meanings set forth below:
- HPLC - high performance liquid chromatography
- BSA- bovine serum albumin from Sigma Chemical Company, St. Louis MO
- g - grams
- sec - seconds
- ms - milliseconds
- mM - millimolar
- DMF - dimethyl formamide
- DMSO - dimethyl sulfoxide
- THF - tetrahydrofuran
- NMR - nuclear magnetic resonance spectroscopy
- TMSCl - tetramethylsilylchloride
- EDAC - 1-ethyl-3-(3-dimethylaminopropyl)-carbodiimide hydrochloride
- EDTA - ethylenediaminetetraacetic acid
- MES - 2-(N-morpholino)ethane sulfonic acid
- MOPS - 3-(morpholino)propane sulfonic acid
- EtOAc - ethyl acetate
- Me - methyl
- OMe - methoxy
- OAc - acetate
- MeOH - methanol
- MS - mass spectrum
- Naphthalocyanine (Nc) - bis (trihexylsilyl)silicon-t-butyl₄-naphthalocyanine
- Phthalocyanine (Pc) - bis(trihexylsilyl)silicon-t-butyl₄-phthatocyanine
- SIAX - succinimidyl 6-((iodoacetyl)amino)hexanoate
- ¹O₂ - singlet oxygen
- SiO₂ - silica gel
- TEA - triethylamine
- THF - tetrahydrofuran
- TCEP - tris-carboxyethyl phosphine
- IHBB - 50 mM KCI, 4 mM MgCl₂, 10 mM trisHCL, 200 µg/ml BSA, pH 8.3
- DPA beads - latex particles dyed with a mixture of thioxene and 9,10-diphenylanthracene
- TAR beads - latex particles dyed with a mixture of thioxene, 1-chloro-9,10-bisphenylethynylanthracene and rubrene
- dopDPA - DPA beads doped with heptadecylbenzene as plasticizer
- dopTAR - TAR beads doped with heptadecylbenzene as plasticizer
- Buffer A - 0.1 M Trisma base, 0.3 M NaCl, 25 mM EDTA, 1 mg/ml dextran T-500, 1 mg/ml BSA, 0.3 % (v/v) HBR-1, 0.05 % kathon, 0.01 % gentamycin sulfate, pH 8.2
- h - hour
- min - minute

### PREPARATION OF REAGENTS

### Synthesis of C-28 thioxene:

To a solution of 4-bromoaniline (30g, 174 mmol) in dry DMF (200 mL) was added 1-bromotetradecane (89.3 mL, 366 mmol) and N,N-diisopropylethylamine (62.2 mL, 357 mmol). The reaction solution was heated at 90°C for 16 hr under argon before being cooled to room temperature. To this reaction solution was again added 1-bromotetradecane (45 mL, 184 mmol) and N,N-diisopropylethylamine (31 mL, 178 mmol) and the reaction mixture was heated at 90°C for another 15 hr. After cooling, the reaction solution was concentrated in vacuo and the residue was diluted with CH₂Cl₂ (400 mL). The CH₂Cl₂ solution was washed with 1N aqueous NaOH (2x), H₂O, and brine, was dried over Na₂SO₄ and was concentrated in vacuo to yield a dark brown oil (about 110g). Preparative column chromatography on silica gel by a Waters 500 Prep LC system eluting with hexane afforded a yellow oil that contained mainly the product (4-bromo-N,N-di-(C₁₄H₂₉)-aniline) along with a minor component 1-bromotetradecane. The latter compound was removed from the mixture by vacuum distillation (bp 105-110°C, 0.6 mm) to leave 50.2g (51%) of the product as a brown oil. To a mixture of magnesium turnings (9.60g, 395 mmol) in dry THF (30 mL) under argon was added dropwise a solution of the above substituted aniline product (44.7g, 79 mmol) in THF (250 mL). A few crystals of iodine were added to initiate the formation of the Grignard reagent. When the reaction mixture became warm and began to reflux, the addition rate was regulated to maintain a gentle reflux. After addition was complete, the mixture was heated at reflux for an additional hour. The cooled supernatant solution was transferred via cannula to an addition funnel and added dropwise (over 2.5 hr) to a solution of phenylglyoxal (11.7g, 87 mmol) in THF (300 mL) at -30°C under argon. The reaction mixture was gradually warmed to 0°C over 1 hr and stirred for another 30 min. The resulting mixture was poured into a mixture of ice water (800 mL) and ethyl acetate (250 mL). The organic phase was separated and the aqueous phase was extracted with ethyl acetate (3x). The combined organic phases were washed with H₂O (2x), brine and were dried over MgSO₄. Evaporation of the solvent gave 48.8g of the crude product as a dark green oily liquid. Flash column chromatography of this liquid (gradient elution with hexane, 1.5:98.5, 3:97, 5:95 ethyl acetate:hexane) afforded 24.7g (50%) of the benzoin product (LSIMS (C₄₂H₆₉NO₂): [M-H]⁺ 618.6, ¹H NMR (250 MHz, CDCl₃) was consistent with the expected benzoin product. To a solution of the benzoin product from above (24.7g, 40 mmol) in dry toluene (500 mL) was added sequentially 2-mercaptoethanol (25g, 320 mmol) and TMSCl (100 mL, 788 mmol). The reaction solution was heated at reflux for 23 hr under argon before being cooled to room temperature. To this was added additional TMSCI (50 mL, 394 mmol); and the reaction solution was heated at reflux for another 3 hr. The resulting solution was cooled, was made basic with cold 2.5N aqueous NaOH and was extracted with CH₂Cl₂ (3x). The combined organic layers were washed with saturated aqueous NaHCO₃ (2x) and brine, was dried over Na₂SO₄ and was concentrated in vacuo to give a brown oily liquid. Preparative column chromatography on silica gel by using a Waters 500 Prep LC system (gradient elution with hexane, 1:99, 2:98 ethyl acetate:hexane) provided 15.5g (60%) of the C-28 thioxene as an orange-yellow oil (LSIMS (C₄₄H₇₁NOS): [M-H]⁺ 661.6, ¹H NMR (250 MHz, CDCl₃) was consistent with the expected C-28 thioxene product 2-(4-(N,N-di-(C₁₄H₂₉)-anilino)-3-phenyl thioxene.

### Synthesis of phthalocyanine sensitizer

Sodium metal, freshly cut (5.0g, 208 mmol), was added to 300 mL of anhydrous methanol in a two-liter, 3-necked flask equipped with a magnetic stirrer, reflux condenser, a drying tube and a gas bubbler. After the sodium was completely dissolved, 4-t-butyl-1,2-dicyanobenzene (38.64g, 210 mmol, from TCI Chemicals, Portland OR) was added using a funnel. The mixture became clear and the temperature increased to about 50°C. At this point a continuous stream of anhydrous ammonia gas was introduced through the glass bubbler into the reaction mixture for 1 hr. The reaction mixture was then heated under reflux for 4 hr. while the stream of ammonia gas continued during the course of the reaction, as solid started to precipitate. The resulting suspension was evaporated to dryness (house vacuum) and the residue was suspended in water (400 mL) and filtered. The solid was dried (60°C, house vacuum, P₂O₅). The yield of the product (1,3-diiminoisoindoline, 42.2g) was almost quantitative. This material was used for the next step without further purification. To a one-liter, three-necked flask equipped with a condenser and a drying tube was added the above product (18g, 89 mmol) and quinoline (200 mL, Aldrich Chemical Company, St. Louis MO). Silicon tetrachloride (11 mL, 95 mmol, Aldrich Chemical Company) was added with a syringe to the stirred solution over a period of 10 minutes. After the addition was completed, the reaction mixture was heated to 180-185°C in an oil bath for 1 hr. The reaction was allowed to cool to room temperature and concentrated HCl was carefully added to acidify the reaction mixture (pH 5-6). The dark brown reaction mixture was cooled and filtered. The solid was washed with 100 mL of water and dried (house vacuum, 60°C, P₂O₅). The solid material was placed in a 1-liter, round bottom flask and concentrated sulfuric acid (500 mL) was added with stirring. The mixture was stirred for 4 hr. at 60°C and was then carefully diluted with crushed ice (2000g). The resulting mixture was filtered and the solid was washed with 100 mL of water and dried. The dark blue solid was transferred to a 1-liter, round bottom flask, concentrated ammonia (500 mL) was added, and the mixture was heated and stirred under reflux for 2 hr., was cooled to room temperature and was filtered. The solid was washed with 50 mL of water and dried under vacuum (house vacuum, 60°C, P₂O₅) to give 12g of product silicon tetra-t-butyl phthalocyanine as a dark blue solid. 3-picoline (12g, from Aldrich Chemical Company), tri-n-butyl amine (anhydrous, 40 mL) and tri-n-hexyl chlorosilane (11.5g) were added to 12g of the above product in a one-liter, three-necked flask, equipped with a magnetic stirrer and a reflux condenser. The mixture was heated under reflux for 1.5 hr. and then cooled to room temperature. The picoline was distilled off under high vacuum (oil pump at about 1 mm Hg) to dryness. The residue was dissolved in CH₂Cl₂ and purified using a silica gel column (hexane) to give 10 g of pure product di-(tri-n-hexylsilyl)-silicon tetra-t-butyl phthalocyanine as a dark blue solid. (LSIMS: [M-H]⁺ 1364.2, absorption spectra: methanol: 674 nm (ε 180,000): toluene 678 nm, ¹H NMR (250 MHz, CDCl₃): δ: -2.4(m,12H), -1.3(m, 12H), 0.2-0.9 (m, 54H), 1.8(s, 36H), 8.3(d, 4H) and 9.6 (m, 8H) was consistent with the above expected product.

### Synthesis of naphthalocyanine sensitizer

### 1. Preparation of 6-t-Butyl-diiminobenz(f)isoindoline (2)

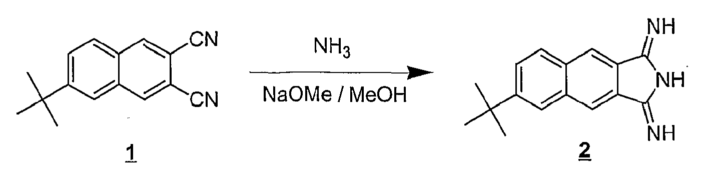

The 6-t-butyl-2,3-dicyanonaphthalene (1) (9.07 g, 38.7 mmol) was suspended in 50 mL MeOH. While stirring under argon, 8.5 mL of 1.05 N NaOMe/MeOH (freshly prepared from Na⁰) was added and the light yellow suspension was bubbled with NH₃ gas for 1 h at ambient temperature. Dilution with an additional 75 ml MeOH did not dissolve the fine suspension. Bubbling with NH₃ was continued at 65° for 2 h until the suspension cleared, and then an additional 1 h. Volatiles were removed by rotary evaporation, and the sticky semi-solid was triturated with 200 mL water. After drying under vacuum (< 1 mmHg) overnight at ambient temperature, the 9.2 g solid was ground by mortar and pestle to a fine green-brown powder. NMR (250 MHz, CDCl₃) showed the absence of the dicyanonaphthalene and was consistent with the desired product. TLC (2:1 MeOH/CH₂Cl₂) was comparable to a sample of nor-butyl diiminobenzisoindoline.

### 2. Preparation of Dichlorosilicon tetra-t-butyl-naphthalocyanine (t-bu₄-NcCl₂) (3)

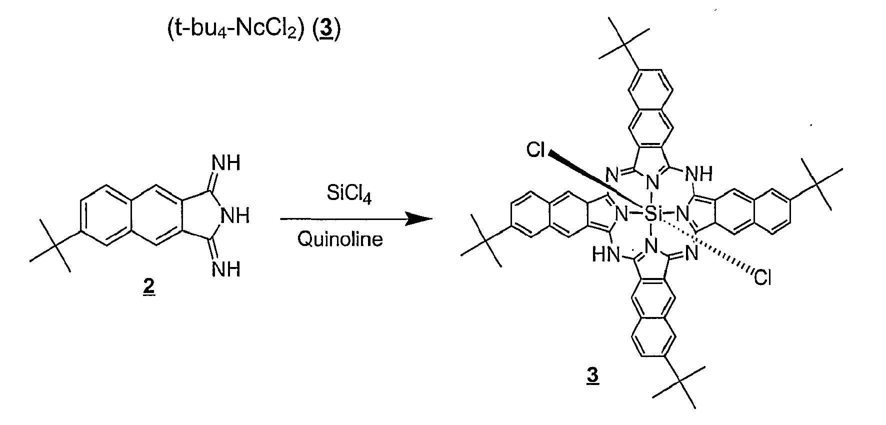

The 6-t-Butyl-diiminobenzisoindoline (**2**) (9.01 g, 35.9 mmol). Prepared as described above, was stirred in 100 mL quinoline. Silicon tetrachloride (4.0 mL, 35 mmol) was added in 1 mL increments at such a rate (~30 min) that the mild exotherm was maintained below 30°. The reaction was then gradually heated by oil bath from 60° to 180°, and stirred under argon for 1 h at that temperature. After cooling to ambient temperature, the reaction was pipetted into two 250 cc polypropylene centrifuge bottles, each containing 150 mL 1:1 H₂O/MeOH. The residue was rinsed with 50 mL of the 1:1 H₂O/MeOH into the two bottles. The bottles were inverted several times to mix the contents, and centrifuged for 10 min at 6K rpm, decanted, and the solid was resuspended in 200 mL 1:1 H₂O/MeOH. The process was repeated 3x, and the final solid was vacuum dried at 60°C/ P₂O₅ affording 8.9 g of the crude product (**3**), which was ground to a green powder by mortar and pestle and used directly in the next step. TLC (2:1 MeOH/CH₂Cl₂) showed the absence of starting material.

### 3. Preparation of Dihydroxysilicon tetra-t-butyl-naphthalocyanine (t-bu₄-Nc(OH)₂) (4)

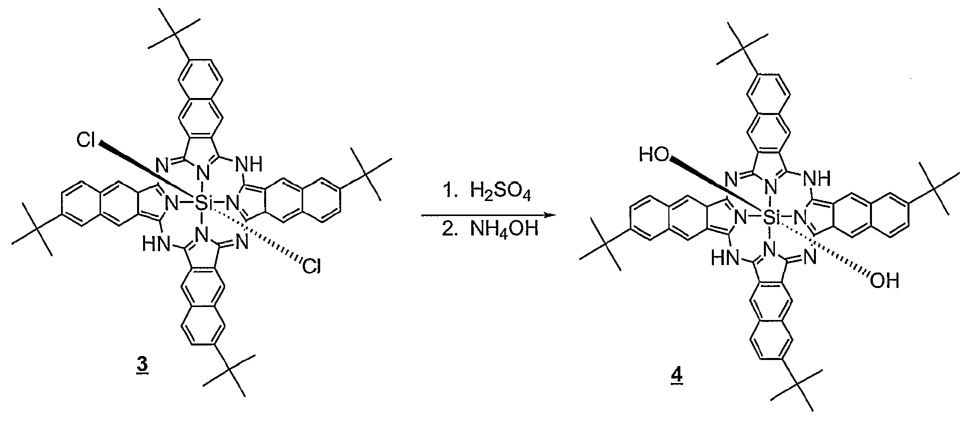

A portion (5.8 g, 5.7 mmol) of the t-bu₄-NcCl₂ (**3**), prepared as described above, was dissolved in 150 mL conc. H₂SO₄. The clear deep purple solution was stirred for 4 h at 60°, cooled and poured into 800 cc ice. The brown suspension was compacted by centrifuge (6K rpm, 6 min), and the solid was washed twice with 200 mL portions of water in each of four 250 cc centrifuge bottles. The final solid was vacuum dried at 60°C/ P₂O₅) affording 6.1 g of black (v. deep reddish brown) solid. This solid.was rinsed from the bottles with 250 mL concentrated NH₄OH into a flask. The deep green suspension was gradually heated to 100°C. After 30 min, frothing subsided, and heating was continued for two hours. After cooling, the suspension was rinsed with 200 mL water into two 250 cc centrifuge bottles. The solid was washed repeatedly by centrifugation (3x, 6K to 12 K rpm) with 150 mL portions of water/bottle. Because of poor compaction, a final centrifugation was done in two 30 cc tubes with 25 mL portions of water at 16 K rpm. The solid was vacuum dried (16 h, 30 mmHg, 60°C, P₂O₅) and weighed (2.822 g). Further drying (3 d, < 1 mmHg, 100°) showed that the partially dried solid still contained 5% water (resulting in reduced yields in some reactions). The identity of the product was confirmed by MS (M⁺ 998).

### 4. Preparation of Bis(tri-n-hexylsilyl)silicon tetra-t-butyl-

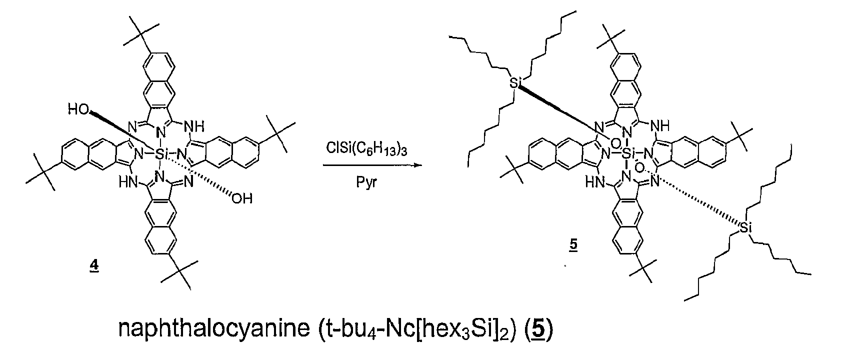

The t-bu₄-Nc(OH)₂ (**4**) (1.61 g, 1.6 mmol), prepared as described above, was dissolved in a solution of 15 mL tri-n-hexylsilylchloride / 30 mL dry pyridine. After bubbling with argon, the solution was heated at 120° for 1 h. Heating the reaction for an additional 2 h did not alter the product distribution as judged by TLC (SiO₂, 10% CH₂Cl₂/hexane). The reaction was cooled to ambient temperature, and the reaction mixture was partitioned between 150 mL hexane and 100 mL water. The mixture was stirred for 30 min until the hexane phase cleared to a deep green color. The aqueous phase was removed and the organic phase was washed twice with 150 mL portions of water, until the final aqueous phase was colorless (pH about 6). The organic phase was filtered (glasswool/funnel), concentrated to about 25 mL, and chromatographed (SiO₂; 5% CH₂Cl₂/hexane). Re-chromatography (Chromatotron: SiO₂ rotor, 0 to 10% CH₂Cl₂/hexane) was necessary to separate the t-butyl isomers. A portion of the major isomer (94 mg) was isolated and used as is for bead dyeing.

### Synthesis of Hydroxypropylaminodextran.

Hydroxypropylaminodextran was prepared by dissolving 100 g of Dextran T-500 (Pharmacia, Uppsala, Sweden) in 500 mL of water in a 3-neck round-bottom flask with a mechanical stirrer and dropping funnel. To the solution was added 45 g sodium hydroxide, 50 mg EDTA, 50 mg NaBH₄, 50 mg hydroquinone, and 200 g N-(2,3-epoxypropyl)phthalimide. The mixture was heated and stirred in a 90°C water bath for two hours. A small aliquot was precipitated three times from methanol and analyzed by NMR. The appearance of a peak at 7.3-7.6 indicated incorporation of phthalimide. The main reaction mixture was precipitated by addition to 3.5 L of methanol, after which solid was collected. The phthalimide protecting group was removed by dissolving the product above in 500 mL of 0.1 M acetate buffer, adding 50 mL of 35% hydrazine, and adjusting the pH to 3.5. The mixture was heated at 80°C for 1 h, the pH was re-adjusted to 3.2, and the mixture was heated for an additional half hour. An aliquot was precipitated three times in methanol. NMR showed that the phthalimide group was no longer present. The reaction mixture was neutralized to pH 8 and stored at room temperature.

The product was purified by tangential flow filtration using a 50,000 molecular weight cut-off filter, washing with water, 0.01 M HCl, 0.01 M NaOH, and finally water. The product solution was concentrated by filtration to 700 mL then lyophilized. Determination of reactive amines using trinitrobenzenesulfonate gave about 1 amine per 16 glucose residues.

### Preparation of phthalocyanine dyed sensitizer beads

The sensitizer beads were prepared by placing 600 mL of carboxylate modified latex (Seradyn) in a three-necked, round-bottom flask equipped with a mechanical stirrer, a glass stopper with a thermometer attached to it in one neck, and a funnel in the opposite neck. The flask had been immersed in an oil bath maintained at 94±1°C. The beads were added to the flask through the funnel in the neck and the bead container was rinsed with 830 mL of ethoxyethanol, 1700 mL of ethylene glycol and 60 mL of 0.1N NaOH and the rinse was added to the flask through the funnel. The funnel was replaced with a 24-40 rubber septum. The beads were stirred at 765 rpm at a temperature of 94±1°C for 40 min.

Silicon tetra-t-butyl phthalocyanine (10.0 g) was dissolved in 300 mL of benzyl alcohol at 60+/-5°C. Eighty-five (85) mL was added to the above round bottom flask through the septum by means of a 100 ml syringe heated to 120+/- 10°C at a rate of 3 mL per min. The remaining phthalocyanine solution was then added similarly. The syringe and flask originally containing the phthalocyanine was rinsed with 40 mL of benzyl alcohol and transferred to a round-bottom flask. After 15 min 900 mL of deionized water and 75 mL of 0.1 N NaOH was added dropwise over 40 min. The temperature of the oil bath was allowed to drop slowly to 40+/- 10°C and stirring was then discontinued. The beads were then filtered through a 43 micron polyester filter and subjected to a Microgon tangential flow filtration apparatus (Microgon Inc., Laguna Hills, CA) using ethanol:water, 100:0 to 10:90, and then filtered through a 43 micron polyester filter.

### Preparation of naphthalocyanine dyed sensitizer beads

A 10 % suspension of carboxylated latex beads (4.4 ml) was mixed with 4.4 mL ethoxyethanol, 8.8 mL ethylene glycol, and 0.44 mL 0.1 N sodium hydroxide solution. Naphthalocyanine (0.475 mg) was dissolved in 0.4 mL benzyl alcohol. One ml of the diluted bead suspension (25 mg beads) was transferred to a 13 x 100 mm glass tube and placed in a heat block maintained at 95 degrees. Two hundred µl of the dye solution was transferred to a separate tube and placed in the heat block. After allowing a few minutes for the temperature to equilibrate, the contents of the tubes were rapidly mixed and heating was continued for an additional 20 minutes. The dyed bead suspension was removed from the heat block and allowed to cool to room temperature at which time it was diluted with 3 ml ethanol and thoroughly mixed. The suspension was then centrifuged to form a pellet of beads. The supernatant was discarded and the pellet was suspended in 50% ethanol in water by sonication. Centrifugation was repeated and the pellet suspended in 10 % ethanol in water. The suspension was centrifuged at a slow speed to pellet a trace of debris from the dyeing procedure. The beads remaining in the supernatant were decanted and stored at 4 degrees C.

### Preparation of hydroxypropylaminodextran coated phthalocyanine sensitizer beads

Hydroxypropylaminodextran solution was prepared at 2 mg/mL in 50 mM MES pH 6. One hundred fifty mg phthalocyanine sensitizer beads in 7.5 mL water was added dropwise to 7.5 mL of the hydroxypropylaminodextran solution while vortexing. One hundred eighty eight µl of EDAC solution (80 mg/ml) in water was added to the coating mixture while vortexing. The mixture was incubated overnight at room temperature in the dark. The mixture was diluted with 12 mL water and centrifuged. The supernatant was discarded and the bead pellet suspended in 40 mL water by sonication. The beads were washed 3 times with water (40 ml per wash) by repeated centrifugation and suspension by sonication. The final pellet was suspended in 5 mL water.

### Preparation of hydroxypropylaminodextran coated naphthalocyanine sensitizer beads

Hydroxypropylaminodextran solution was prepared at 10 mg/mL in 50 mM MES pH 6. Twenty mg of the naphthalocyanine sensitizer beads in 1 mL 10 % ethanol in water was slowly added to 1 mL of the hydroxypropylaminodextran solution while vortexing. Two mg EDAC dissolved in 0.2 mL water was added to the coating mixture while vortexing. Following an overnight incubation at room temperature, the mixture was centrifuged, the supernatant discarded, and the bead pellet suspended in 2 mL water by sonication. The water wash was repeated 2 times by centrifugation and the final bead pellet suspended in 1 mL water.

### Preparation of hydroxyproiaylaminodextran coated dopTAR chemiluminescer beads

A 10% suspension of carboxylated latex beads (120 mL) was heated to 93 °C in a three-neck round bottom flask, then mixed with 166 mL ethoxyethanol, 336 mL ethylene glycol, and 12 mL of 0.1 M NaOH. A mechanical stirrer and thermometer were added, and the mixture was brought to 95°C with stirring, then stirred for an additional 40 minutes. In a separate flask, 2.45 g of C-28 thioxene, 191.8 mg of 2-chloro-9,10-bis(phenylethynyl)anthracene, and 323.9 mg of rubrene were dissolved in 264 mL of ethoxyethanol and heated to 95°C with stirring until dissolved. The dye solution was poured into the bead suspension and stirred for 20 min at 95°C, then allowed to cool slowly to about 47°C and filtered through a 43 µm polyester filter to remove any debris generated during the dyeing procedure. The beads were washed by tangential flow filtration using a Microgon apparatus with a P698/4 filter. After priming of the system with wash solvent (1:2 v/v ethoxyethanol and ethylene glycol), the dyed bead mixture was added, concentrated to about 20 mg/mL, then washed with 6 liters of wash solvent and 4.8 L of 10% v/v ethanol in water adjusted to pH 10 with NaOH. The TAR beads were concentrated to about 50 mg/mL during the wash, then stored at 4 °C protected from light.

A plasticizer was incorporated into the beads to enhance the rate of decay of luminescence. A mixture was prepared containing 250 µL of n-heptadecylbenzene, 20 mL of ethanol, and 0.5 g of hydroxypropylaminodextran dissolved in 25 mL of 50 mM MES pH 6. The mixture was heated to 80°C in an oil bath and stirred vigorously to disperse the plasticizer. A second mixture containing 40 mL of dyed TAR beads from above (diluted to 25 mg/mL in 10% ethanol) and 30 mL of 50 mM MES pH 6 was also heated to 80°C. The two mixtures were combined and left stirring at 80°C overnight.

After cooling, the beads were separated, by pipette, from a small amount of excess plasticizer that floated on top of the bead suspension. EDAC (200 mg) in 3 mL of water was added, and the mixture was stirred at room temperature for 2 h. The mixture was then centrifuged to recover the beads. The bead pellet was suspended by sonication and washed with three 40 mL portions of water by alternating centrifugation and suspension by sonication. The final bead pellet was then suspended in about 35 mL water.

### Preparation of hydroxypropylaminodextran coated dopDPA chemiluminescer beads

A 10 % suspension of carboxylated latex beads (10 ml) was mixed with 10 mL ethoxyethanol, 20 mL ethylene glycol, and 1 mL 0.1 N sodium hydroxide solution in a 250 mL Erlenmeyer flask with stir bar. The flask was clamped in an oil bath maintained at 95 degrees C. In a separate flask 200 mg C28 thioxene and 30 mg 9,10-diphenylanthracene was dissolved in 20 ml ethoxyethanol and brought to 95 degrees C. The contents of the flasks were rapidly mixed and heating at 95 degrees C was continued for 90 min. The flask was removed from the oil bath and allowed to cool to room temperature. After cooling, the mixture was diluted with 60 mL ethanol, thoroughly mixed, and the dyed beads collected by centrifugation. The supernatant was discarded and the bead pellet suspended in 20 mL ethanol by sonication. Centrifugation was repeated and the beads washed with a second 20 mL portion of ethanol. Following a final centrifugation, the beads were suspended in 40 mL water.

A plasticizer was incorporated into the beads to enhance the rate of decay of luminescence. The dyed DPA beads from above were placed in a 125 mL Erlenmeyer flask, fitted with stir bar, and placed in an 80 degree C oil bath. Forty mL ethanol was placed in a 50 mL Erlenmeyer flask and 300 mg heptadecylbenzene (HB) plasticizer added. The solution was equilibrated in the oil bath and then rapidly added to the bead suspension with vigorous mixing. The mixture was incubated in the 80 degree C oil bath for 90 min.

While the bead/plasticizer mixture was incubating, a solution of hydroxypropylaminodextran was prepared. Three hundred twenty mg of hydroxypropylaminodextran was dissolved in 16 ml 50 mM MES pH 6 and the solution warmed to 80 degrees C in the oil bath. The solution was then added all at once to the bead suspension with vigorous stirring. The flask was fitted with a water condenser to minimize evaporative loses and heating at 80 degrees C was continued overnight.

The mixture was cooled to room temperature and 50 mg EDAC in water added with stirring. Stirring at room temperature was continued for 2 h, at which time, 20 mL ethanol was added and the suspension centrifuged. The supernatant was discarded and the beads suspended in 40 ml 50% aqueous ethanol by sonication. Centrifugation was repeated and the beads suspended in 0.5 M NaCl by sonication. Following a final centrifugation the beads were suspended in 10% aqueous ethanol, 50 mM NaCl.

### Preparation of A₂₄ oligonucleotide coated phthalocyanine sensitizer particles (Pc-A₂₄)

Sixty five mg of hydroxypropylaminodextran coated phthalocyanine sensitizer beads were suspended in 5 mL 50 mM MOPS pH 7. A 10 % (w/v) SIAX solution was prepared in DMF and 77 µl added to the bead suspension while vortexing. The mixture was incubated at room temperature for 90 min in the dark and then a second 77 µl aliquot of SIAX solution added and the mixture incubated for an additional 60 min. The suspension was centrifuged and the supernatant discarded. The bead pellet was suspended in 6 mL water by sonication and the centrifugation repeated. The pellet was suspended in 6.5 mL water and stored at 4 degrees C.

In preparation for oligonucleotide coupling, the beads were centrifuged, the supernatant was discarded, and 1.34 mL coupling buffer added to the pellet. Coupling buffer consists of the following mixture: (900 µL 0.2 M borate, 2 mM EDTA pH 9 and 333 µL of 0.4 M borate pH 9.45 and 1000 µL of 2 M Na₂SO₄) which had been degassed and saturated with argon. Nine µL of 10% Tween 20 detergent was added to the coupling buffer mixture after degassing and saturating with argon.

5' A₂₄ (SEQ ID NO:1) oligonucleotide modified at the 3' end with -PO₂OCH₂CH₂CH₂SSCH₂CH₂CH₂OH was dissolved in water and the concentration determined by optical density at 260 nm. Using the extinction coefficient supplied by the vendor the concentration was found to be 915.8 nmoles/mL. Approximately twelve nmoles of oligonucleotide per mg of beads was used for the coupling procedure.

Seven hundred sixty µL of oligonucleotide solution was placed in a centrifuge tube and 76 µL of 2.5 M NaOAc pH 5.3 added. One hundred forty seven µL of 20 mM TCEP in water was added to the oligonucleotide solution and the mixture incubated 1 h at room temperature in the dark. Four volumes of 200 proof ethanol was added to the mixture to precipitate the reduced oligonucleotide. Precipitation was facilitated by placing the mixture in a -20 degree C freezer for 1 hour. The precipitate was collected by centrifugation and then dissolved in 495 µL 5 mM Na₂HPO₄, 2 mM EDTA pH 6 that had been degassed and saturated with argon.

The oligonucleotide solution was then added to the bead pellet under coupling buffer and the mixture was sonicated to suspend the beads. The suspension was incubated at 37 degrees C for 23 h.

Residual iodo groups on the iodoaminodextran coat were capped by reaction with mercaptoacetic acid. The bead suspension was centrifuged and the supernatant removed. The pellet was suspended by sonication in 5 mL of 10 mM mercaptoacetic acid in 0.4 M borate pH 9.45 and the mixture incubated at 37 degrees C for 1 h. The beads were recovered by centrifugation and suspended in 5 mL blocking buffer: (0.1 M NaCl, 0.17 M glycine, 10 mg/mL BSA, 0.1% Tween 20, 1 mM EDTA pH 9.2 sterile filtered and 50 µL/mL Calf Thymus DNA added). The mixture was incubated for 3 h at 37 degrees C. Following centrifugation, the beads were washed twice by centrifugation with 5 mL Buffer A per wash. The final pellet was suspended in 6 mL IHBB buffer and incubated at 95 degrees C for 90 min. After cooling, the beads were centrifuged, the supernatant was discarded, and the pellet was suspended in 6 ml of equal volumes of 0.125 M NaOAc pH 5 and 30% hydrogen peroxide solution. Incubate at 37 degrees for 2.5 hours. The mixture was centrifuged, the supernatant discarded, and the beads washed 3 times by centrifugation with storage buffer (50 mM KCI, 10 mM tris, 4 mM EDTA, 0.2% acetylated BSA pH 8.2) using 5 mL buffer per wash. The final pellet was suspended by sonication in 6 mL storage buffer and stored at 4 degrees protected from light.

### Preparation of (AGTA)₆ oligonucleotide coated dopDPA chemiluminescer particles (dopDPA-(AGTA)₆)

The oligonucleotide coated beads were prepared by a procedure similar to that described above for the preparation of Pc-A₂₄ beads. However, only the sensitizer beads were treated with peroxide. 5'-(AGTA)₆-3' (SEQ ID NO:2) oligonucleotide modified at the 3' end with -PO₂OCH₂CH₂CH₂SSCH₂CH₂CH₂OH was employed.

### Preparation of (ATAG)₆ oligonucleotide coated naphthalocyanine sensitizer particles (Nc-(ATAG)₆)

The oligonucleotide coated beads were prepared by a procedure similar to that described above for the preparation of Pc-A₂₄ beads. However, only the sensitizer beads were treated with peroxide. 5'-(ATAG)₆-3' (SEQ ID NO:3) oligonucleotide modified at the 3' end with -PO₂OCH₂CH₂CH₂SSCH₂CH₂CH₂OH was employed.

### Preparation of A₂₄ oligonucleotide coated dopTAR chemiluminescer particles (dopTAR-A₂₄)

The oligonucleotide coated beads were prepared by a procedure similar to that described above for the preparation of Pc-A₂₄ beads. However, only the sensitizer beads were treated with peroxide. 5' A₂₄ (SEQ ID NO:1) oligonucleotide modified at the 3' end with -PO₂OCH₂CH₂CH₂SSCH₂CH₂CH₂OH was employed.

### Preparation of (AGTA)₆ oligonucleotide coated phthalocyanine sensitizer particles (Pc-(AGTA)₆)

The oligonucleotide coated beads were prepared by a procedure similar to that described above for the preparation of Pc-A₂₄ beads. However, only the sensitizer beads were treated with peroxide. 5'-(AGTA)₆-3' (SEQ ID NO:2) oligonucleotide modified at the 3' end with -PO₂OCH₂CH₂CH₂SSCH₂CH₂CH₂OH was employed.

### Oligonucleotide Probe and Linker Sequences

RL-2 probe:
   5'- CTC ACA GTC AGA AAT TGG AGT GTA CTT ACT TAC TTA CTT ACT X - 3' (SEQ ID NO:4)
RL-3 probe:
   5' - TTT TTT TTT TTT TTT TTT TTA GAC TTT TTC TAT TCG CAG CGC X - 3' (SEQ ID NO:5)
RF-3 probe:
   5' - GAC AGT GTA GAT AGA TGA CAG TCG CTA TCT ATC TAT CTA TCT AT X - 3' (SEQ ID NO:6)
AL-1 linker:
   5' - ACT GTC ATC TAT CTA CAC TGT TTT TGC GCT GCG AAT AGA AAA AGT C - 3' (SEQ ID NO:7)
SP-1 linker: 
5' – B TA CTT ACT TAC TTA CTT ACT TAC TGA ATG GGT TAG AGT GCA TCC
AGT GCT ATC TAT CTA TCT ATC TAT CTA T – 3'   (SEQ ID NO:8)
SO linker: 
5’ – CTC CAA TTT CTG ACT GTG AGT TTT TGC GCT GCG AAT AGA AAA AGT
CT – 3’          (SEQ ID NO:9)
CL-1 linker 
5' – B TA CTT ACT TAC TTA CTT ACT TAC TGA ATG GGA TAG AGT GCA TCC
AGT GTT TTT TTT TTT TTT TTT TTT TTT T – 3'     (SEQ ID NO:10)
HA-1 linker 
5' – CTA TCT ATC TAT CTA TCT ATC TAT GAA TGG GAT AGA GTG CAT CCA
GTG TTT TTT TTT TTT TTT TTT TTT TTT – 3'          (SEQ ID NO:11)
X = 3' terminus blocked with C₇ amine (CH₂CH(CH₂OH)CH₂CH₂CH₂CH₂NH₃)
B = biotin

### Example 1

### Assay according to Scheme 1

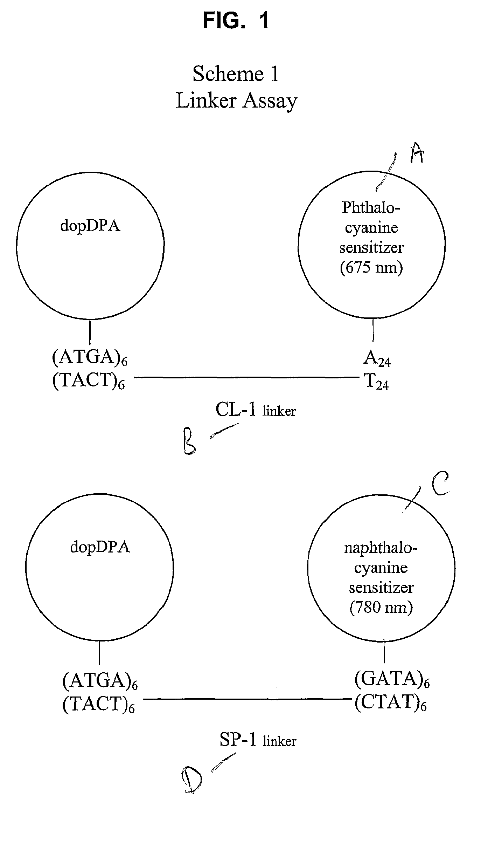

Oligonucleotide linkers having end sequences complementary to the oligonucleotide sequences attached to the beads were employed and were synthesized by Oligos Etc. The beads set forth above, namely, ^{5'}(ATAG)^{3'}₆- Nc became bound to the complementary tail, namely, ^{5'}(CTAT)^{3'}₆, of the SP-1 linker identified above as SEQ ID NO:8. Similarly, the other end of the linker was complementary to a chemiluminescer bead as shown in Scheme 1 (Fig. 1). In the presence of linker, the two beads were brought together in a simple "sandwich" type assay and, due to their close proximity and the transfer of singlet oxygen, generated a signal that was directly proportional to the linker concentration.

### Protocol 1:

| Master mix | per assay | master mix |
|---|---|---|
| water | 39 µl | 1.56 ml |
| 10X IHBB | 5 µl | 200 µl |
| dopDPA-(AGTA)₆ (5 mg/ml) | 0.5 µl | 20 µl |
| Pc-A₂₄ (5 mg/ml) | 0.25 µl | 10 µl |
| Nc-(ATAG)₆ (5 mg/ml) | 0.25 µl | 10 µl |
| | 45 µl | 1.8 ml |

Linker working solutions were freshly diluted from frozen concentrates into DNAse free water containing 2 µg/ml tRNA.
CL-1 linker working solutions: 1 nM, 500 pM, 100 pM, 50 pM, 10 pM, 5 pM, and 0 pM (water)
SP-1 linker working solutions: 1 nM, 500 pM, 100 pM, 50 pM, 10 pM, 5 pM, and 0 pM (water)
Mixed linkers (CL-1/SP-1) working solutions: 500/500 pM, 500/50 pM, 50/500 pM, 50/50 pM

Forty-five (45) µL master mix was placed in a PCR tube and 5 µL working solution was added and mixed. The material was topped with 20 µL mineral oil and incubated in a PCR cycler for 2 min. at 95°C, 15 min. at 50°C, and 60 min. at 37°C. The tubes were transferred to a heat block maintained at 37°C while taking signal readings. Each sample was read three times.

The three readings were averaged and a standard curve was constructed. From the standard curve the concentrations of the linkers in the mixed linker samples were determined.

### Assay:

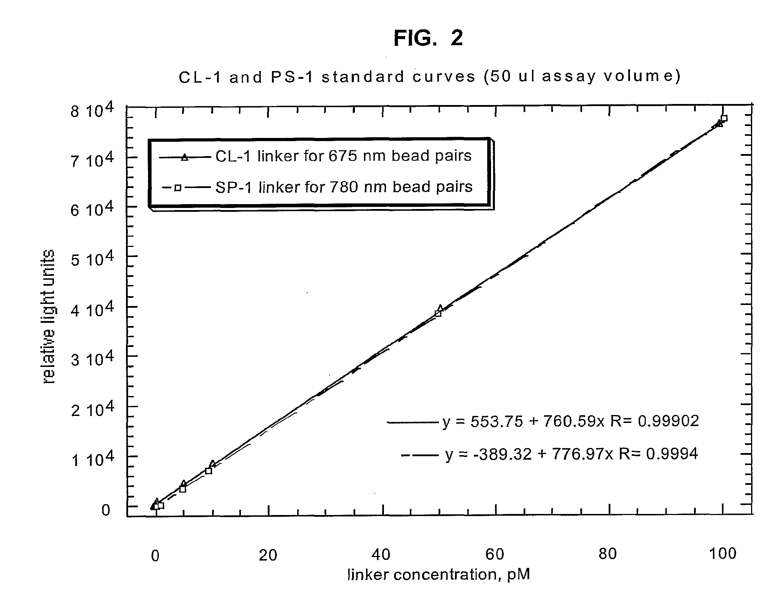

Standard curves for the linkers CL-1 and SP-1 were generated using Protocol 1. Equal aliquots (typically 45 µL) of a "master mix" composed of the two sensitizer beads and the chemiluminescer bead in IHBB buffer were placed in each of several PCR tubes. Then, an aliquot (typically 5 µL) of serially diluted linker standard was added followed by mineral oil (20 µL). Linker working solutions were freshly prepared from a concentrated stock solution. A similar set of standards was prepared using the second linker. Following incubation, the samples were read with the dual laser reader and the results are summarized in Table 1. Each sample was first illuminated with the 675 nm laser followed by the 780 nm laser. The reading was repeated three times and averaged. The average was corrected by subtracting the reading obtained when no linker was present in the sample. Then, the corrected averages were used to construct linker standard curves (Fig. 2). Similarly, a sample set was prepared using the three-bead "master mix" and linker mixtures. Following incubation, the samples were read as before and the results are summarized in Table 2. From the corrected average of the readings, the linker mixtures were quantitated by reference to the standard curves. The quantitated values, in parenthesis, are also shown in Table 2 and may be compared to the known concentrations of the mixed linkers shown in the first column.

**Table 1**

| Raw data from reader (each sample was read three times and averaged) | | | | | | | | | |
|---|---|---|---|---|---|---|---|---|---|
| linker conc. | CL-1 linker for 675 nm bead pairs | | | | | SP-1 linker for 780 nm bead pairs | | | |
| | 675 nm | 0 linker | 780 nm | 0 linker | | 675 nm | 0 linker | 780 nm | 0 linker |
| | laser | corrected | laser | Corrected | | laser | corrected | laser | corrected |
| 100 pM | 74833 | | 1718 | | | 418 | | 79066 | |
| | 73360 | | 1756 | | | 367 | | 78564 | |
| | 78526 | | 1773 | | | 322 | | 74926 | |
| average | **75573** | ***75268*** | **1749** | ***850*** | | **369** | ***31*** | **77519** | ***76516*** |
| 50 pM | 42372 | | 1386 | | | 390 | | 44468 | |
| | 39892 | | 1368 | | | 286 | | 40955 | |
| | 42314 | | 1385 | | | 309 | | 38602 | |
| average | **41526** | ***41221*** | **1380** | ***481*** | | **328** | ***0*** | **41342** | ***40339*** |
| 10 pM | 9135 | | 1042 | | | 437 | | 8148 | |
| | 8667 | | 968 | | | 310 | | 7173 | |
| | 9088 | | 1003 | | | 289 | | 6364 | |
| average | **8963** | ***8658*** | **1004** | ***105*** | | **345** | *7* | **7228** | ***6225*** |
| 5 pM | 4383 | | 1078 | | | 350 | | 4210 | |
| | 4154 | | 983 | | | 285 | | 3813 | |
| | 4392 | | 977 | | | 221 | | 3336 | |
| average | **4310** | ***4005*** | **1013** | **114** | | **285** | ***0*** | **3786** | **2783** |
| 1 pM | 1228 | | 981 | | | 410 | | 1691 | |
| | 1152 | | 905 | | | 347 | | 1556 | |
| | 1089 | | 896 | | | 335 | | 1395 | |
| average | **1156** | ***851*** | 927 | ***28*** | | **364** | ***26*** | **1547** | ***544*** |
| 0.5 pM | 863 | | 1285 | | | 366 | | 1312 | |
| | 828 | | 1254 | | | 256 | | 1247 | |
| | 758 | | 1191 | | | 254 | | 1148 | |
| average | **816** | ***511*** | **1243** | ***344*** | | **292** | ***0*** | **1236** | **233** |
| 0.0 pM | 359 | | 917 | | | 408 | | 1051 | |
| | 263 | | 890 | | | 269 | | 990 | |
| | 294 | | 890 | | | 336 | | 967 | |
| average | **305** | ***0*** | **899** | ***0*** | | **338** | ***0*** | **1003** | ***0*** |
| air | 92 | | 786 | | | 92 | | 786 | |
| | 96 | | 780 | | | 96 | | 780 | |
| | 96 | | 768 | | | 96 | | 768 | |
| average | **95** | | **778** | | | **95** | | **778** | |

**Table 2**

| Raw data from variable linker mixtures (each sample was read three times and averaged) | | | | | | |
|---|---|---|---|---|---|---|
| CL-1/SP-1 ratio | 675 nm laser | 0 linker corrected | | 780 nm laser | 0 linker corrected | |
| 50/50 pM | 38116 | | | 41168 | | |
| | 36851 | | | 39195 | | |
| | 40186 | | | 36456 | | |
| average | **38384** | ***38079*** | ***(=49.3pM)*** | **38940** | ***37937*** | ***(=49.3 pM)*** |
| 50/5 pM | 44265 | | | 5194 | | |
| | 42957 | | | 4637 | | |
| | 46629 | | | 4111 | | |
| average | **44617** | ***44312*** | ***(=57.5 pM)*** | **4647** | ***3644*** | ***(=5.2 pM)*** |
| 5/50 pM | 4404 | | | 40744 | | |
| | 3919 | | | 38473 | | |
| | 4197 | | | 35301 | | |
| average | **4173** | ***3868*** | ***(=4.4 pM)*** | **38173** | ***37170*** | ***(=48.3 pM)*** |
| 5/5 pM | 4346 | | | 4642 | | |
| | 4024 | | | 3982 | | |
| | 4231 | | | 3681 | | |
| average | **4200** | ***3895*** | ***(=4.4 pM)*** | **4102** | ***3099*** | ***(=4.5 pM)*** |

| | | | | | | |
|---|---|---|---|---|---|---|
| Values in ***(bold italics)*** were obtained from the standard curves for linker assay. A single linker was employed. | | | | | | |

### Example 2

### Assay according to Scheme 2

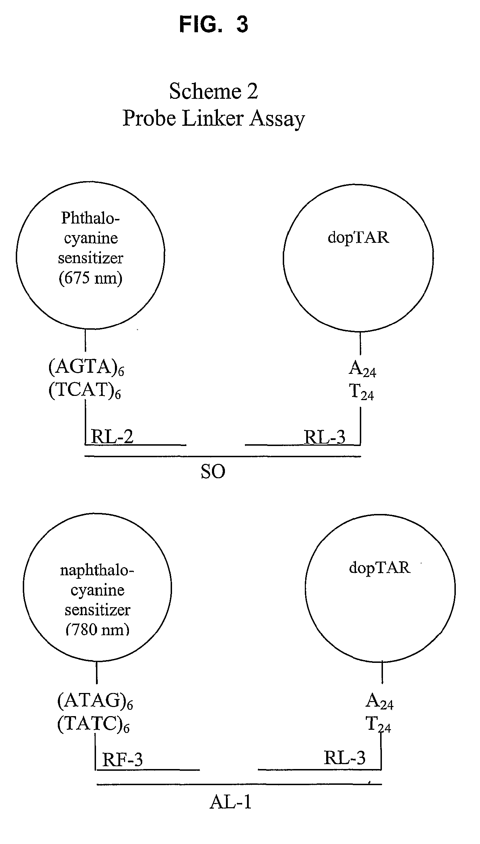

Oligonucleotide probes and linkers were synthesized by Oligos Etc. In this assay, the probes had complementary sequences such that one end of the probe became bound to an oligonucleotide on a bead and the other end became bound to a linker. The linkers have one end that was common for the probes and became bound to the chemiluminescer beads. The other end of the linker was specific for the probe that became bound either to the phthalocyanine sensitizer or the naphthalocyanine sensitizer according to Scheme 2 (Fig. 3). The probes were present in excess over the linker but were less abundant than the total amount of complementary oligonucleotides on the beads such that each bead became decorated with several probes and no free probe was left in solution. In the presence of linker, the probe decorated beads became bound to the linker in a "sandwich" type assay that generated a signal in direct proportion to the linker concentration.

### Protocol 2:

| Master mix | per assay | master mix |
|---|---|---|
| water | 37.372 µl | 1.856 ml |
| 10X IHBB | 5 µl | 250 µl |
| dopTAR-A₂₄ (5 mg/ml) | 0.50 µl | 25 µl |
| Pc-(AGTA)₆ (5 mg/ml) | 0.25 µl | 12.5 µl |
| Nc-(ATAG)₆ (5 mg/ml) | 0.25 µl | 12.5 µl |
| probe RL-3, 1 µM | 0.625 µl | 31.25 µl |
| (binds dopTAR to SO and AL-1 linkers) | | |
| probe RL-2, 1 µM | 0.625 µl | 31.25 µl |
| (binds PS-(AGTA)₆ to SO linker) | | |
| probe RF-3, 1 µM | 0.625 µl | 31.25 µl |
| (binds NS-(ATAG)₆ to AL-1 linker) | | |

| | | |
|---|---|---|
| SO linker working stocks: 1000, 500, 100, 50, 10, 5, and 0 pM in water. AL-1 linker working stocks: 1000, 500, 100, 50, 10, 5, and 0 pM in water. | | |

Forty-five (45) µl of master mix was placed in each of several snap-cap PCR tubes. Five (5) µl of linker working stock was added and the mixtures topped with 20 µl mineral oil. Using a PCR cycler, the material was incubated 2 min at 95°C, 15 min at 50°C, and 90 min at 37°C. Each sample was read once.

### Assay:

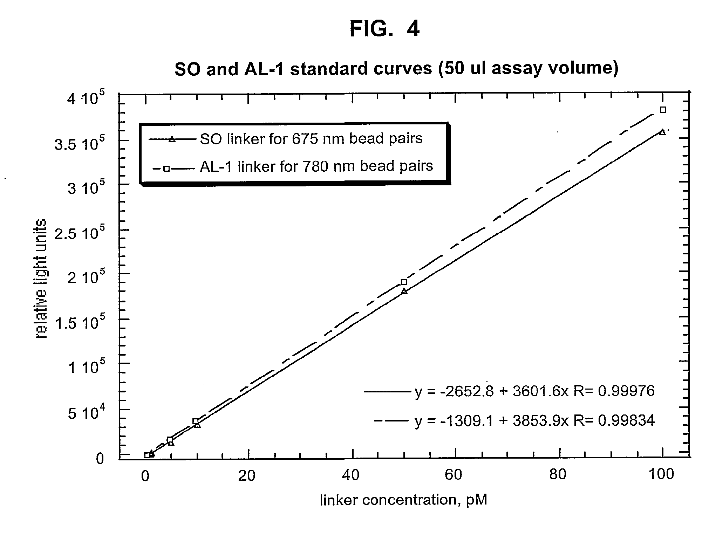

Standard curves for the linkers SO and AL-1 were prepared using Protocol 2. A master-mix consisting of both sensitizers, a single chemiluminescer, and three probes in IHBB buffer was prepared. Aliquots (45 µl) of the master mix were placed in PCR tubes followed by an aliquot (5 µl) of serially diluted linker standard and mineral oil (20 µl). Duplicate samples were set up for both linkers. Following incubation, the samples were read with a dual laser reader. (Table 3). The duplicates were averaged and the averages corrected by subtracting the reading obtained when no linker was present in the sample. The corrected averages were then used to construct standard curves for the linkers (Fig. 4). Similarly, a sample set was prepared from "master mix" and a mixture of the two linkers. Following incubation, the samples were read as before and the results are summarized in Table 4. From the corrected average of the readings, the linker mixtures were quantitated using the linker standard curves. The quantitated values, in parenthesis, are also shown in Table 4 and may be compared to the known concentrations of the mixed linkers shown in the first column.

**Table 3**

| Raw data from reader (duplicates averaged) | | | | | | | | | |
|---|---|---|---|---|---|---|---|---|---|
| pM linker | SO linker for 675 nm bead pairs | | | | | AL-1 linker for 780 nm bead pairs | | | |
| conc. | 675 nm | 0 linker | 780 nm | 0 linker | | 675 nm | 0 linker | 780 nm | 0 linker |
| | laser | corrected | laser | corrected | | laser | corrected | laser | corrected |
| 100 pM | 357681 | | 3368 | | | 3864 | | 376232 | |
| | 358282 | | 3152 | | | 4079 | | 380424 | |
| average | **357982** | ***356657*** | **3260** | ***1391*** | | **3972** | ***2647*** | **378328** | ***376459*** |
| 50 pM | 180225 | | 3317 | | | 2497 | | 207210 | |
| | 182250 | | 2137 | | | 2475 | | 213618 | |
| average | **181238** | ***179913*** | **2727** | ***858*** | | **2486** | ***1161*** | **210414** | ***208545*** |
| 10 pM | 31082 | | 1834 | | | 1327 | | 29851 | |
| | 35822 | | 2591 | | | 1388 | | 32730 | |
| average | **33452** | ***32127*** | **2213** | ***344*** | | **1358** | ***33*** | **31291** | ***29422*** |
| 5 pM | 11560 | | 1915 | | | 1253 | | 15362 | |
| | 10227 | | 1654 | | | 1415 | | 17156 | |
| average | **10894** | ***9569*** | **1785** | ***-84*** | | **1334** | ***11*** | **16259** | ***14390*** |
| 1 pM | 3126 | | 2252 | | | 1894 | | 4997 | |
| | 2915 | | 2221 | | | 1275 | | 3822 | |
| average | **3021** | ***1696*** | **2237** | **368** | | **1585** | ***260*** | **4410** | ***2541*** |
| 0.5 pM | 2764 | | 2548 | | | 1595 | | 3388 | |
| | 2202 | | 2551 | | | 1186 | | 2659 | |
| average | **2483** | ***1131*** | **2550** | ***681*** | | **1391** | ***66*** | **3024** | **1155** |
| 0.0 pM | 1303 | | 1749 | | | 1305 | | 1998 | |
| | 1210 | | 2094 | | | 1478 | | 1634 | |
| average | **1257** | ***0*** | **1922** | ***0*** | | **1392** | ***0*** | **1816** | ***0*** |
| air | 179 | | 885 | | | 179 | | 976 | |
| | 191 | | 948 | | | 169 | | 941 | |
| average | **185** | | **917** | | | **174** | | **959** | |

**Table 4**

| Raw data from variable linker mixtures (duplicates averaged) | | | | | | |
|---|---|---|---|---|---|---|
| SO/AL-1 | 675 nm | 0 linker | | 780 nm | 0 linker | |
| ratio | laser | corrected | | laser | corrected | |
| 50/50 pM | 189721 | | | 207813 | | |
| | 195089 | | | 212006 | | |
| average | **192405** | ***191080*** | ***(=53.8 pM)*** | **209910** | ***208041*** | ***(=54.3 pM)*** |
| 50/5 pM | 180290 | | | 12893 | | |
| | 179343 | | | 12991 | | |
| average | **179817** | ***178492*** | ***(=50.3 pM)*** | **12942** | ***11073*** | ***(=3.2 pM)*** |
| 5/50 pM | 12179 | | | 204797 | | |
| | 12403 | | | 213823 | | |
| average | **12291** | ***10966*** | ***(=3.8 pM)*** | **209310** | ***207441*** | ***(=54.2 pM)*** |
| 5/5 pM | 11629 | | | 12801 | | |
| | 10960 | | | 12491 | | |
| average | **11295** | ***9970*** | ***(=3.5 pM)*** | **12646** | ***10777*** | ***(=3.1 pM)*** |

| | | | | | | |
|---|---|---|---|---|---|---|
| Values in ***(bold italics)*** were obtained from the standard curves for probe linker assay | | | | | | |

### Example 3

### Assay for CL-1 and HA-1

The protocol for this assay was essentially the same as that described for the assay in Example 1 above. Briefly, aliquots (typically 45 µL) of a master mix containing 3.75 µg of each of the sensitizer beads was added to 200 µL PCR tubes. Target polynucleotide (5 µL) (CL-1, HA-1 or a combination thereof) at appropriate concentrations was added to the master mix. Twenty (20) µL of mineral oil was then added to each tube. The mixtures in the tubes were subjected to thermal cycling as follows: 95°C for 3 min, 50°C for 15 min and 37°C for 120 min. The tubes were illuminated and read.

After subtraction of background (no target polynucleotide in the reaction tubes), the signal crossover from each oligonucleotide linker into the other channel was determined. The signals were then corrected to remove crossover signal from the other channel. The corrected signal was used to plot standard curves for the two oligonucleotide linkers as well as to determine the effect of the presence of one oligonucleotide linker on the quantification of the other linker (see Table 5 and Table 6). Table 5 shows the calculated CL-1 concentration in the presence of HA-1. Table 6 shows the calculated HA-1 concentration in the presence of CL-1.

**Table 5**

| | Input HA-1 (pM) | | | | |
|---|---|---|---|---|---|
| Input CL-1 (pM) | 0.05 | 0.25 | 2.50 | 25.00 | 100.00 |
| 0.05 | 0.04 | 0.05 | 0.05 | 0.04 | 0.04 |
| 0.25 | 0.23 | 0.23 | 0.22 | 0.23 | 0.21 |
| 2.5 | 2.4 | 2.4 | 2.3 | | |
| 25.0 | 24.4 | 24.3 | | 23.8 | |
| 100 | 95.3 | 96.6 | | | 94.4 |

**Table 6**

| | Input CL-1 (pM) | | | | |
|---|---|---|---|---|---|
| Input HA-1 (pM) | 0.05 | 0.25 | 2.50 | 25.00 | 100.00 |
| 0.05 | 0.04 | 0.03 | 0.04 | 0.05 | 0.05 |
| 0.25 | 0.24 | 0.24 | 0.24 | 0.24 | 0.25 |
| 2.5 | 2.5 | 2.4 | 2.4 | | |
| 25.0 | 23.8 | 24.0 | | 24.4 | |
| 100 | 99.7 | 101.3 | | | 95.5 |

### Example 4

### Amplification and quantitation of a Mycobacterium tuberculosis DNA

The amplification and detection of Genomic *Mycobacterium tuberculosis* (M. tb) target DNA was carried out using PCR and the reagents for the signal producing system described above. Detection was accomplished with one chemiluminescent compound and two sensitizers.

Genomic *Mycobacterium tuberculosis* (M. tb) target DNA (GenBank Accession NO. Y14045NID) was obtained from C. Green, SRI International, Menlo Park, CA. The region used for amplification and detection was in the insertion sequence 6110 (IS6110) which is present in multiple copies per M. tb genome (~ 10 copies/genome). All the PCR primers including those used for the generation of the 494 bp internal control (IC) amplicon are shown below. The sequence numbers are IS6110 sequence. The 24 base IC specific sequence introduced in the IC amplicon is underlined.

The forward primers were:
ZL-3 18 mer 2645-2662
   5' CCGTCCCGCCGATCTCGT (SEQ ID NO:12)
LB-3 18 mer 2833-2850
   5' CGATCGAGCAAGCCATCT 3' Tm 66.8 (SEQ ID NO:13)

The primers for introducing 24 base Q sequence into IS6110 region were:
LH-1
   5' GACAGTGTAGATAGATGACAGTCGCATCGATCCGGTTCAGCG (SEQ ID NO:14)
LH-2
   5'CGACTGTCATCTATCTACACTGTCGGTGGATAACGTCTTTCAC (SEQ ID NO:15)

The reverse primers were:
ZL-4 20 mer 2952-2971
   5' GACGGTTGGATGCCTGCCTC (SEQ ID NO:16)
LH-4 22 mer 3117-3138
   5' ACTGGTAGAGGCGGCGATGGTT (SEQ ID NO:17)

The Internal Control (IC) amplicon for M.tb IS6110 was constructed as follows. The 24 base internal sequence was introduced using standard PCR procedures. The outer primers used were ZL-3 and LH-4. The internal primers LH-1 and LH-2 used for replacement of 24 base WT sequence by 24 base IC sequences are shown above.

PCR amplicons were made using primer pairs ZL-3 and LH-2 and a second amplicon was generated using LH-1 and LH-4. The expected amplicons were 278 and 240 base pairs, respectively. The two amplicons generated were then annealed, extended and re-amplified using the external primers ZL-3 and LH-4. The amplification product was run on a 1.5% Agarose gel and the 494-bp amplicon was excised from the gel. The IC amplicon was re-amplified with the outer primers ZL-3 and LH-4 and the 494 bp product was gel purified. Finally, the IC amplicon was produced by PCR amplification quantified by using gel electrophoresis and known ds DNA standards. The IC amplicon was also quantified using limit dilution of the target followed by PCR amplification. The IC amplicon was purified using a Microcon-100 (Amicon Inc., Beverly, Massachusetts) to remove of unincorporated primers and nucleotides; aliquots were stored frozen at -20°C.

The following probes were used for detection:
The common probe was:
   ZL-5 20 mer 2869-2887
      5' (T)₂₀ GCGTACTCGACCTGAAAGAC (SEQ ID NO:18)
This probe binds to the common sequence present on both the WT and IC targets as well as to the common (A) ₂₄ sequence on labeled particles. In the assay, the probe binds to the (A)₂₄ sequence on the single DopTAR chemiluminescer particle (DopTAR-(A)₂₄₎.

The WT specific probe was:
ZN-1 20 mer 2901-2920
   5' ACGGATAGGGGATCTCAGTA (TACT)₅ (SEQ ID NO:19)
This probe binds to the WT specific sequence and also to a phthalocyanine sensitizer particle (Pc-(AGTA)₆) via the (TACT)₅ probe tail.

The IC specific Probe was:
IC 24 mer 2901-2920 on IC amplicon only.
   5' GACAGTGTAGATAGATGACAGTCG (CTAT)₅ (SEQ ID NO:20)
This is an IC specific probe and binds to the 24 base engineered sequence present on the IC amplicon. In the assay this probe also binds to the naphthalocyanine sensitizer particle (Nc-(ATAG)₆) via the (CTAT)₅ tail present on the probe.

All oligonucleotides were synthesized by Oligos Etc. In addition, all the probe sequences used were 3'- amino blocked according to known procedures and gel purified to prevent probe participation during the PCR amplification.

M.tb target and IC DNA was amplified by PCR using the following protocol. In the assay using two sensitizers, the reaction mixture consisted of 200 µM nucleotide tri-phosphates (dNTPs) (Pharmacia Biotech.), 250 nM primers LB-3 and ZL-4, 5 units cPfu (Stratagene), 25 nM each of probes ZL-5, ZN-1 and IC, 3.75 µg of common chemiluminescer particle (DopTAR -(A₂₄)), and 2.5 µg of each of the specific sensitizer particles (Pc-(AGTA)₆ and Nc-(ATAG)₆) in a 50 µl reaction. The reaction was carried out in a buffer consisting of 10 mM Tris-HCL, 50 mM KCI, 4 mM MgCl₂, 0.2 mg/ml acetylated BSA (Gibco BRL). Finally, after addition of the target, 20 µl of mineral oil was added to each of the reactions. PCR thermal cycling was done as follows: 95°C (3 min.) initial denaturation, {95°C (20 sec.), 63°C (1 min.), 73°C (1 min.)} x 36 cycles, followed by 75°C (5 min.). Immediately following the PCR amplification, the double stranded amplicon was denatured 95°C (2 min.), probes were annealed to the target at 50°C (15 min.). Then, the probes were allowed to bind to the label particles at 37°C for 60 minutes.

A series of known amounts of M.tb target DNA was amplified in the presence of constant amount of IC DNA added to the sample. Amplification of M.tb target and IC target with primers LB-3 and ZL-4 results in a 139-bp amplicon. The common probe ZL-5 binds to both the WT Mt.b as well as IC amplicons on one hand and to the chemiluminescent particle via the probe tail. However, the specific probe ZN-1 binds to WT amplicon and to phthalocyanine particle (Pc) while, the IC probe binds specifically to IC amplicon and to the naphthalocyanine particle (Nc). After carrying out the amplification and annealing the reagents to the amplicons generated, the chemiluminescent signal generated by the particle pairs formed was read with a reader as mentioned above. The WT signal (Pc) was obtained by illuminating the tubes with 675-nm laser for 1.0 seconds followed by reading the chemiluminescent signal for 1 second. This was followed by 10-second delay to allow the entire chemiluminescer signal from the first particle pair to decay completely. The IC signal (Nc) was read 6 times with one-second illumination and one-second read with a 780-nm laser. The background (no target) signal was subtracted from the readings. After removing the crosstalk signal, corrected signal was obtained and is shown in Table 7 (below).

**Table 7**

| M.tb genomes (WT input) | IC | WT corrected* signal (Pc)(RLU) | IC corrected** signal (Nc)(RLU) | WT genomes Derived (Q WT) |
|---|---|---|---|---|
| 1.2E+01 | 0 | 1160470 | 1477 | |
| 4.00E+03 | 0 | 1260836 | -1605 | |
| 0 | 1.20E+02 | -56 | 1143140 | |
| 0 | 4.00E+04 | 61 | 1263188 | |
| 5.00E+01 | 2.00E+04 | 232015 | 1136556 | 45 |
| 5.00E+01 | 2.00E+04 | 203468 | 1113000 | 39 |
| 2.00E+02 | 2.00E+04 | 673804 | 949025 | 315 |
| 2.00E+02 | 2.00E+04 | 661124 | 906437 | 331 |
| 2.00E+02 | 2.00E+04 | 652938 | 893193 | 332 |
| 2.00E+03 | 2.00E+04 | 941123 | 632355 | 1392 |
| 2.00E+03 | 2.00E+04 | 917323 | 640118 | 1282 |
| 2.00E+03 | 2.00E+04 | 938149 | 651096 | 1297 |
| 2.00E+04 | 2.00E+04 | 1130373 | 264144 | 17847 |
| 2.00E+04 | 2.00E+04 | 1149292 | 273789 | 16973 |
| 5.00E+04 | 2.00E+04 | 1161752 | 178213 | 54901 |
| 5.00E+04 | 2.00E+04 | 1154424 | 163623 | 68142 |

| | | | | |
|---|---|---|---|---|
| * Corrected Pc signal (WT) = [S-Bp_{c} minus (S-B_{Nc} x 0.0022)] wherein S-B is Signal-Background. The correction factor 0.0022 reflects 0.22% of Nc crossover signal into the Pc signal channel. ** Corrected Nc signal (IC) = [S-B_{Nc} minus (S-Bp_{c}) x 0.0047) wherein S-B is Signal-Background. The correction factor 0.0047 reflects 0.47% cross-over of Pc crossover signal into the Nc signal channel | | | | |

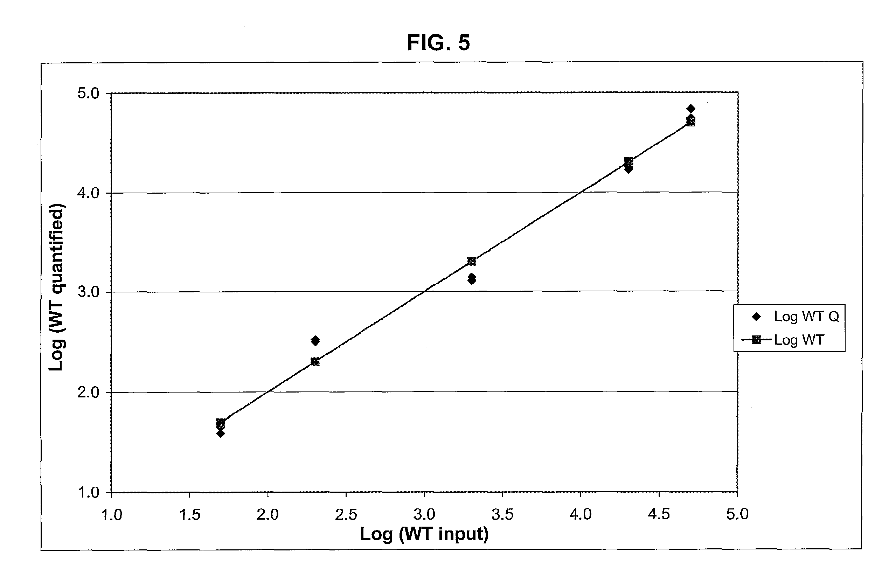

The log of WT derived (log WT Q) was plotted against the log of M. tb genomes added per reaction (log WT input). Log WT Q was derived from the formula: Log (WT Q) = v(log(IC/(WT+IC))+w+log(WT/(WT+IC)) where WT & IC are corrected signals from each bead, v ideally has a value of -1 and w ideally has a value of log(IC targets/per reaction). However, v and w values were derived from a fit to a standard curve for the specific combination of probes, sensitizer and chemiluminescent reagents and assay conditions. The results are shown in FIG. 5.

The above discussion includes certain theories as to mechanisms involved in the present invention. These theories should not be construed to limit the present invention in any way, since it has been demonstrated that the present invention achieves the results described.

Although the foregoing invention has been described in some detail by way of illustration and example for purposes of clarity and understanding, it will be obvious that certain changes or modifications may be practiced within the scope of the appended claims.

## Claims

1. A method for determining the presence or relative amounts of two or more components in a medium, said method comprising :
a) providing in combination (1) a medium suspected of containing said components, (2) at least two sensitizer reagents, said sensitizer reagents being capable of generating a product upon activation, said sensitizer reagents being distinguishable on the basis of activation, and (3) at least one reactive reagent activatable by said product, wherein the combination of sensitizer reagent and reactive reagent allows differential detection of said components, and
b) differentially activating said sensitizer reagents and detecting the amount of signal generated corresponding to the activation of said reactive reagent by said product, the amount thereof being related to the amount of each of said components in said medium.

2. A method for determining the presence or relative amounts of two or more components in a medium, said method comprising :
a) providing in combination (1) a medium suspected of containing said components, (2) at least two sensitizer reagents, said sensitizer reagents being capable of generating singlet oxygen and being distinguishable on the basis of activation, and (3) at least one reactive reagent activatable by singlet oxygen, wherein the combination of sensitizer reagents and reactive reagents allows differential detection of said components, and
b) differentially activating said sensitizer reagents and detecting the amount of signal generated as a result of the activation of said reactive reagent, the amount thereof being related to the amount of each of said components in said medium.

3. A method according to Claim 2 wherein said reactive reagent is a chemiluminescent composition.

4. A method according to Claim 2 wherein multiple reactive reagents are employed.

5. A method according to Claim 2 wherein one of said sensitizer reagents or said reactive reagent is associated with a first specific binding pair (sbp) member and wherein said first sbp member is capable of binding to said component or to a second sbp member to form a complex related to the amount of said component.

6. A method according to Claim 5 wherein said first sbp member is capable of binding to said component and wherein a second sbp member that is associated with the other of said sensitizer reagents or said reactive reagent is capable of binding with said component.
